(19) **Europäisches Patentamt European Patent Office Office européen des brevets**

(11) **EP 3 573 949 B1**

(12) **EUROPEAN PATENT SPECIFICATION**

(45) Date of publication and mention
of the grant of the patent:
**13.10.2021 Bulletin 2021/41**

(51) Int Cl.:
*C07C 209/16* *(2006.01)*   *C07D 295/023* *(2006.01)*
*C07C 211/10* *(2006.01)*   *C07C 211/14* *(2006.01)*

(21) Application number: **17811895.6**

(22) Date of filing: **29.11.2017**

(86) International application number:
**PCT/EP2017/080813**

(87) International publication number:
**WO 2018/099966 (07.06.2018 Gazette 2018/23)**

(54) **PROCESS FOR THE CONVERSION OF MONOETHANOLAMINE TO ETHYLENEDIAMINE EMPLOYING A NANOCRYSTALLINE ZEOLITE OF THE MOR FRAMEWORK STRUCTURE**

VERFAHREN ZUR UMWANDLUNG VON MONOETHANOLAMIN ZU ETHYLENDIAMIN MITTELS EINES NANOKRISTALLINEN ZEOLITHS DER MOR-RAHMENWERKSTRUKTUR

PROCÉDÉ DE CONVERSION DE LA MONOÉTHANOLAMINE EN ÉTHYLÈNEDIAMINE UTILISANT UNE ZÉOLITE NANOCRISTALLINE DE LA STRUCTURE CHARPENTÉE DE MOR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2016 EP 16201340**

(43) Date of publication of application:
**04.12.2019 Bulletin 2019/49**

(73) Proprietor: **BASF SE**
**67056 Ludwigshafen am Rhein (DE)**

(72) Inventors:
• **PARVULESCU, Andrei-Nicolae**
**67056 Ludwigshafen am Rhein (DE)**
• **GORDILLO, Alvaro**
**69123 Heidelberg (DE)**
• **SCHROETER, Marie Katrin**
**67056 Ludwigshafen am Rhein (DE)**
• **MELDER, Johann-Peter**
**67056 Ludwigshafen am Rhein (DE)**
• **BECHTEL, Juergen**
**69123 Heidelberg (DE)**
• **HEIDEMANN, Thomas**
**67056 Ludwigshafen am Rhein (DE)**
• **SCHUNK, Stephan A.**
**69123 Heidelberg (DE)**
• **MÜLLER, Ulrich**
**67056 Ludwigshafen am Rhein (DE)**

(74) Representative: **BASF IP Association**
**BASF SE**
**GBI-C006**
**67056 Ludwigshafen (DE)**

(56) References cited:
**EP-A2- 0 252 424   US-B2- 7 687 423**

**Description**

TECHNICAL FIELD

The present invention relates to a process for the conversion of 2-aminoethanol to ethane-1,2-diamine and/or linear polyethylenimines of the formula $H_2N-[CH_2CH_2NH]_n-CH_2CH_2NH_2$ wherein $n \geq 1$, said process employing a zeolitic material having the MOR framework structure comprising $YO_2$ and $X_2O_3$, wherein Y is a tetravalent element and X is a trivalent element, said zeolitic material displaying an average particle size of the zeolitic material along the 002 axis of the crystallites in the range of from $5 \pm 1$ nm to $55 \pm 8$ nm as determined by powder X-ray diffraction.

INTRODUCTION

Mordenite (MOR) zeolites are a class of large pore zeolites with 1 dimensional 12 membered ring (MR) channels and intersecting 8-MR channels (side pockets). Al substitution into the neutral silicate framework creates a charge imbalance compensated by cations (e.g., O-H group that act as Brønsted acids). These class of zeolites was previously described for various types of chemical transformations like Friedel-Crafts type reactions, isomerization, carbonilation and amination processes. The MOR zeolites synthesis was also described following different reaction pathways like template free-synthesis (in the absence of an organic pore forming agent), templated synthesis or post-modification like de-alumination reaction. Thus, WO 2014/135662 A relates to the carbonilation of dimethyl ether (DME) with syngas to methylacetate, wherein the MOR zeolite employed is preferably made with a tetraethylammonium bromide (TEABr) template. US 7,605,295 concerns a transalkylation process employing small crystal MOR zeolites with a mean crystallite length parallel to the direction of 12 MR pores of preferably 50 nm, wherein the zeolite is made with a TEABr template. Similarly, US 7,687,423 B2 relates to the synthesis of crystallites having the MOR framework structure with a mean crystallite length parallel to the direction of the 12-ring channels of 60 nm or less as well as to their use in the transalkylation of aromatics. Grundner, Sebastian et al. in Nat. Commun. 2015, Vol. 6, article number 7546 concerns Cu-MOR as selective/active catalyst for methane oxidation to methanol involving a specific Cu ion exchange method starting from $Cu(OAc)_2$ without consecutive calcination.

Mordenite based catalysts are also known for the synthesis of ethylene amines by gas-phase amination mainly of monoethanol amine. Thus, US 4,918,233 reports on the use of a rare-earth doped MOR for the monoethanol amine (MEOA) gas-phase amination with 80 % selectivity to EDA at 26 % MEOA conversion. CN 1962058 A relates to the gas-phase synthesis of ethylenediamine the amination of ethanolamine using a Mordenite catalyst containing one of Zr, Nb, Mo, or Sn in combination with Zn or Fe. JP H0687797 A and JP H07247245 A respectively relate to a process for the gas-phase reaction of ammonia and monothanolamine to ethylenediamine with the use of a dealuminated Mordenite catalyst. Modification of the mordenite zeolite with P for production of EDA and piperazine derivates from MEOA is also described in CN 101215239 B. CN 101406845 A describes an H-mordenite amination catalyst and its preparation. CN 102974393 A relates to a method for regeneration of modified zeolite molecular sieve amination catalysts. CN 103007984 A claims a method for manufacturing amination catalysts. CN102233272A and CN102190588A a process for preparing EDA through catalytic amination of monoethylene glycol (MEG).

In addition to these, the inaugural thesis "Heterogeneous Transition Metal Catalyzed Amination of Aliphatic Diols" from Achim Fischer, Diss. ETH No 12978, 1998, discusses zeolite catalyzed processes for the conversion of monoethyleneglycol and monoethanolamine to ethylenediamine, respectively. WO 2009/083580 A1 relates to a process for the production of ethylene amines from the amination of ethylene oxide, ethylene glycol, or ethanolamine using a sulfonated tetrafluoroethylene based fluoropolymer-copolymer (Nafion) as a catalyst. US 4918233 concerns the production of ethylenediamine from monoethanolamine and ammonia with the use of a dealuminated Mordenite catalyst. It is noted that all of the aforementioned processes concern amination processes which are conducted in the liquid phase and require the use of high pressure.

Finally, CN 101215239 A concerns the joint preparation of ethylene diamine and aminoethyl piperazine, wherein the process involves the use of a phosphorous modified mordenite catalyst.

Despite the available methods for the amination of monoethanolamine, there remains a need for the provision of improved processes employing catalysts which not only display a higher activity but also allow for an increased selectivity with respect to the amination products, and in particular with respect to ethylene diamine.

DETAILED DESCRIPTION

It was therefore the object of the present invention to provide a process for the conversion of 2-aminoethanol to ethane-1,2-diamine and/or linear polyethylenimines of the formula $H_2N-[CH_2CH_2NH]_n-CH_2CH_2NH_2$ wherein $n \geq 1$ permitting to obtain higher yields of ethane-1,2-diamine and/or linear polyethylenimines of the formula $H_2N-[CH_2CH_2NH]_n-CH_2CH_2NH_2$ wherein $n \geq 1$, at higher conversion rates of the monoethanolamine precursor compound. Thus, it has surprisingly found that by using a catalyst comprising a zeolitic material having the MOR framework structure, wherein said zeolitic material displays an average particle size along the 002 axis of the crystallites in the range of from $5 \pm 1$ nm to $55 \pm 8$ nm as determined by powder X-ray diffraction, a process for the conversion of 2-aminoethanol to ethane-1,2-diamine and/or linear polyethylenimines of the formula $H_2N\text{-}[CH_2CH_2NH]_n\text{-}CH_2CH_2NH_2$ wherein $n \geq 1$ is provided permitting not only to achieve higher conversion rates, but which is furthermore more selective towards ethane-1,2-diamine and/or the aforementioned linear polyethylenimines.

Therefore, the present invention relates to a process for the conversion of 2-aminoethanol to ethane-1,2-diamine and/or linear polyethylenimines of the formula $H_2N\text{-}[CH_2CH_2NH]_n\text{-}CH_2CH_2NH_2$ wherein $n \geq 1$ comprising

(i) providing a catalyst comprising a zeolitic material having the MOR framework structure comprising $YO_2$ and $X_2O_3$, wherein Y is a tetravalent element and X is a trivalent element;
(ii) providing a gas stream comprising 2-aminoethanol and ammonia;
(iii) contacting the catalyst provided in (i) with the gas stream provided in (ii) for converting 2-aminoethanol to ethane-1,2-diamine and/or linear polyethylenimines,

wherein $n$ preferably ranges from 1 to 8, more preferably from 1-5, more preferably from 1-4, more preferably from 1-3, more preferably from 1-2, wherein more preferably $n = 1$;
wherein the average particle size of the zeolitic material along the 002 axis of the crystallites is in the range of from $5 \pm 1$ nm to $55 \pm 8$ nm as determined by powder X-ray diffraction.

As regards the average particle size of the zeolitic material having the MOR framework structure along the 002 axis of the crystallites as determined by powder X-ray diffraction, no particular restrictions apply according to the present invention with respect to its determination. According to the present invention, it is however preferred that the values for the average particle size of the zeolitic material having the MOR framework structure along the 002 axis of the crystallites is determined according to the method disclosed in US 7,687,423 B2, in particular as described in col. 8, lines 25-48 of said document. It is, however, further preferred according to the present invention that the values for the average particle size of the zeolitic material having the MOR framework structure along the 002 axis of the crystallites as determined by powder X-ray diffraction is determined according to the method described in the experimental section of the present application, wherein the values are determined based on the X-ray diffraction data by fitting the diffracted peak width using the software TOPAS 4.2, wherein instrumental broadening is considered during the peak fitting using the fundamental parameter approach as described in TOPAS 4.2 Users Manual (Bruker AXS GmbH, Ostliche Rheinbrückenstr. 49, 76187 Karlsruhe, Germany) leading to a separation of the instrumental from the sample broadening, the sample contribution being determined using a single Lorentzian profile function as defined in the following equation:

$$\beta = \lambda \, / \, (L \cdot \cos\theta)$$

where $\beta$ is the Lorentzian full width at half maximum (FWHM), $\lambda$ is the X-ray wavelength of the CuKa radiation used, L is the crystallite size, and $\theta$ is the half the scattering angle of the peak position. According to said preferred method, the crystallite size of the 002 reflection is determined in a refinement of the local data surrounding the 002 reflection, from $21°$ to $24.2°(2\theta)$, wherein single peaks with varying crystallite sizes model the surrounding reflections, the data being collected in the Bragg-Brentano geometry from $2°$ to $70°$ $(2\theta)$, using a step size of $0.02°(2\theta)$.

Regarding the average particle size of the zeolitic material having the MOR framework structure along the 002 axis of the crystallites in the range of from $5 \pm 1$ nm to $55 \pm 8$ nm as determined by powder X-ray diffraction, the skilled person readily understands which process parameters to vary to obtain zeolitic material within all of said range. US 7,605, 295 B1 in column 2, lines 8 to 13 and lines 45 to 47 discloses a UZM aggregate material comprising globular aggregates of crystallites having a MOR framework type having an average crystal size along the 002 axis of the crystallites of about 60 nm or less, preferably about 50 nm or less.

Furthermore, US 7,687,423 B2 in the examples describes methods for preparing zeolitic material having the MOR framework structure along the 002 axis of the crystallites, wherein in example 1 thereof UZM-14-A and UZM-14-B were prepared having an average crystal size along the 002 axis of the crystallites of 47 and 50 nm respectively. Furthermore, in example 3 of US 7,687,423 B2 additional UZM-14 samples were prepared with slight variations to the parameters discussed in example 1 thereof, such that material was prepared having an average crystal size along the 002 axis of the crystallites in the range of from 40.6 to 50.4 nm. Additionally, US 7,687,423 B2 highlights that the prior art material from Zeolist and Tosoh has an average crystal size along the 002 axis of the crystallites of greater than $55 \pm 8$ nm.

Furthermore, the additional examples and comparative examples herein provide further guidance for the skilled person to obtain zeolitic material within all of the above said range.

According to the present invention, it is preferred that the average particle size of the zeolitic material along the 002 axis of the crystallites as determined by powder X-ray diffraction is in the range of from $10 \pm 1$ nm to $53 \pm 8$ nm, more preferably from $15 \pm 2$ nm to $50 \pm 5$ nm, more preferably from $18 \pm 2$ nm to $48 \pm 5$ nm, more preferably from $20 \pm 2$ nm to $45 \pm 5$ nm, more preferably from $23 \pm 2$ nm to $43 \pm 4$ nm, more preferably from $25 \pm 3$ nm to $40 \pm 4$ nm, more preferably from $28 \pm 3$ nm to $38 \pm 4$ nm, and more preferably from $30 \pm 3$ nm to $35 \pm 4$ nm. It is particularly preferred according to the present invention that the particle size of the zeolitic material along the 002 axis of the crystallites as determined by powder X-ray diffraction is in the range of from $32 \pm 3$ nm to $34 \pm 3$ nm.

It is alternatively preferred that the average particle size of the zeolitic material along the 002 axis of the crystallites as determined by powder X-ray diffraction is in the range of from $25 \pm 3$ nm to $41 \pm 4$ nm, preferably from $26 \pm 3$ nm to $40 \pm 4$ nm, more preferably from $27 \pm 3$ nm to $39 \pm 4$ nm, more preferably from $28 \pm 3$ nm to $38 \pm 4$ nm, more preferably from $29 \pm 3$ nm to $37 \pm 4$ nm, more preferably from $30 \pm 3$ nm to $36 \pm 4$ nm, more preferably of from $31 \pm 3$ nm to $35 \pm 4$ nm, and more preferably from $32 \pm 3$ nm to $34 \pm 3$ nm.

It is alternatively preferred that the average particle size of the zeolitic material along the 002 axis of the crystallites as determined by powder X-ray diffraction is in the range of from $38 \pm 4$ nm to $54 \pm 8$ nm, preferably from $39 \pm 4$ nm to $53 \pm 8$ nm, more preferably from $40 \pm 4$ nm to $52 \pm 5$ nm, more preferably from $41 \pm 4$ nm to $51 \pm 5$ nm, more preferably from $42 \pm 4$ to $50 \pm 5$ nm, more preferably from $43 \pm 4$ nm to $49 \pm 5$ nm, more preferably from $44 \pm 4$ nm to $48 \pm 5$ nm, more preferably from $45 \pm 5$ nm to $47 \pm 5$ nm.

It is alternatively preferred that the average particle size of the zeolitic material along the 002 axis of the crystallites as determined by powder X-ray diffraction is in the range of from $39 \pm 4$ nm to $55 \pm 8$ nm, preferably from $40 \pm 4$ nm to $54 \pm 8$ nm, more preferably from $41 \pm 4$ nm to $53 \pm 8$ nm, more preferably from $42 \pm 4$ nm to $52 \pm 5$ nm, more preferably from $43 \pm 4$ nm to $51 \pm 5$ nm, more preferably from $44 \pm 4$ nm from $50 \pm 5$ nm, more preferably from $45 \pm 5$ nm to $49 \pm 5$ nm, more preferably from $46 \pm 5$ nm to $48 \pm 5$ nm.

It is alternatively preferred that the average particle size of the zeolitic material along the 002 axis of the crystallites as determined by powder X-ray diffraction is in the range of from $45 \pm 5$ nm to $55 \pm 8$ nm, preferably from $46 \pm 5$ nm to $54 \pm 8$ nm, more preferably from $47 \pm 5$ nm to $53 \pm 8$ nm, more preferably from $48 \pm 5$ nm to $52 \pm 8$ nm, more preferably from $49 \pm 5$ nm to $51 \pm 5$ nm.

As regards the values of the average particle size of the primary crystallites of the zeolitic material along the 002 axis of the crystallites as determined by powder X-ray diffraction, it is noted that according to the present invention said values are to be understood as containing the following deviation depending on the dimension of the primary crystallites along the 002 axis, said deviation being indicated with "$\pm$" following the given value:

| | |
|---|---|
| >100-200 nm: | 20% (e.g. $\pm$ 30 nm for 150 nm) |
| >50-100 nm: | 15% (e.g. $\pm$ 15 nm for 100 nm) |
| >5-50 nm: | 10% (e.g. $\pm$ 5 nm for 50 nm) |
| 2-5 nm: | 20% (e.g. $\pm$ 1 nm for 5 nm) |

It is preferred according to the present invention that the average particle size of the primary crystallites of the zeolitic material as determined by powder X-ray diffraction is in the range of from $5 \pm 1$ nm to $100 \pm 15$ nm, preferably the average particle size of the primary crystallites is in the range of from $10 \pm 1$ nm to $90 \pm 14$ nm, more preferably from $20 \pm 2$ nm to $85 \pm 13$ nm, more preferably from $30 \pm 3$ nm to $80 \pm 12$ nm, more preferably from $35 \pm 4$ nm to $75 \pm 11$ nm, more preferably from $40 \pm 4$ nm to $70 \pm 11$ nm, more preferably from $45 \pm 5$ nm to $65 \pm 10$ nm, and more preferably from $50 \pm 5$ nm to $65 \pm 10$ nm. It is particularly preferred according to the present invention that the average particle size of the primary crystallites of the zeolitic material as determined by powder X-ray diffraction is in the range of from $55 \pm 8$ nm to $65 \pm 10$ nm. As regards the values of the average particle size of the primary crystallites of the zeolitic material as determined by powder X-ray diffraction, it is noted that according to the present invention said values are to be understood as containing the following deviations depending on the dimension of the primary crystallites, said deviation being indicated with "$\pm$" following the given value:

| | |
|---|---|
| >100-200 nm: | 20% (e.g. $\pm$ 30 nm for 150 nm) |
| >50-100 nm: | 15% (e.g. $\pm$ 15 nm for 100 nm) |
| >5-50 nm: | 10% (e.g. $\pm$ 5 nm for 50 nm) |
| 2-5 nm: | 20% (e.g. $\pm$ 1 nm for 5 nm) |

As regards the average particle size of the primary crystallites of the zeolitic material having the MOR framework structure as determined by powder X-ray diffraction, no particular restrictions apply according to the present invention with respect to its determination. According to the present invention, it is however preferred that the values for the average particle size of the primary crystallites of the zeolitic material having the MOR framework structure as determined by powder X-ray diffraction is determined according to the Scherrer equation. It is, however, further preferred according to the present invention that the values for the average particle size of the primary crystallites of the zeolitic material having the MOR framework structure as determined by powder X-ray diffraction is determined according to the method described in the experimental section of the present application, wherein the values are determined based on the X-ray diffraction data by fitting the diffracted peak width using the software TOPAS 4.2, wherein instrumental broadening is considered during the peak fitting using the fundamental parameter approach as described in TOPAS 4.2 Users Manual (Bruker AXS GmbH, Ostliche Rheinbrückenstr. 49, 76187 Karlsruhe, Germany) leading to a separation of the instrumental from the sample broadening, the sample contribution being determined using a single Lorentzian profile function as defined in the following equation:

$$\beta = \lambda \, / \, (L \cdot \cos\theta)$$

where $\beta$ is the Lorentzian full width at half maximum (FWHM), $\lambda$ is the X-ray wavelength of the CuKa radiation used, L is the average particle size of the primary crystallites, and $\theta$ is the half the scattering angle of the peak position, the data being collected in the Bragg-Brentano geometry from 2° to 70° ($2\theta$), using a step size of 0.02°($2\theta$).

According to the present invention, there is no particular restriction as to the amount of 2-aminoethanol in the gas stream provided in (ii) and contacted with the catalyst in (iii). Thus, by way of example, the gas stream provided in (ii) and contacted with the catalyst in (iii) may contain 2-aminoethanol in an amount in the range of from 0.1 to 10 vol.-%, preferably from 0.5 to 5 vol.-%, more preferably from 1 to 4.5 vol.-%, more preferably from 1.5 to 4 vol.-%, more preferably from 2 to 3.7 vol.-%, more preferably from 2.5 to 3.5 vol.-%, more preferably from 2.7 to 3.3 vol.-%. It is, however, particularly preferred according to the present invention that the gas stream provided in (ii) and contacted with the catalyst in (iii) contains 2-aminoethanol in an amount in the range of from 2.9 to 3.1 vol.-%.

As regards the ammonia content in the gas stream provided in (ii) and contacted with the catalyst in (iii), no particular restrictions apply such that any conceivable amount of ammonia may be chosen for conducting the inventive process. Thus, by way of example, the gas stream provided in (ii) and contacted with the catalyst in (iii) may contain ammonia in an amount in the range of from 5 to 90 vol.-%, preferably from 10 to 80 vol.-%, more preferably from 20 to 70 vol.-%, more preferably from 25 to 60 vol.-%, more preferably from 30 to 50 vol.-%, more preferably from 35 to 45 vol.-%, and more preferably from 37 to 43 vol.-%. It is, however, particularly preferred according to the present invention that the gas stream provided in (ii) and contacted with the catalyst in (iii) contains ammonia in an amount in the range of from 39 to 41 vol.-%.

According to the present invention, there is in principle no restriction as to the ammonia: 2-aminoethanol molar ratio in the gas stream provided in (ii) and contacted with the catalyst in (iii), provided that the molar ratio is in the range of from 1 to 45. Thus, by way of example, the ammonia : 2-aminoethanol molar ratio in the gas stream provided in (ii) and contacted with the catalyst in (iii) may be in the range of from 2 to 35, preferably from 4 to 30, more preferably from 6 to 25, more preferably from 8 to 20, and more preferably from 10 to 16. It is, however, particularly preferred according to the present invention that the ammonia: 2-aminoethanol molar ratio in the gas stream provided in (ii) and contacted with the catalyst in (iii) is in the range of from 12 to 14.

According to the present invention, there is in principle no restriction as to the content of hydrogen in the gas stream provided in (ii) and contacted with the catalyst in (iii). Thus, by way of example, the gas stream provided in (ii) and contacted with the catalyst in (iii) may further contain hydrogen in an amount in the range of from 0.1 to 70 vol.-%, preferably from 0.5 to 50 vol.-%, more preferably from 1 to 40 vol.-%, more preferably from 5 to 35 vol.-%, more preferably from 10 to 30 vol.-%, more preferably from 15 to 25 vol.-%, and more preferably from 17 to 23 vol.-%. It is, however, particularly preferred according to the present invention that the gas stream provided in (ii) and contacted with the catalyst in (iii) contains hydrogen in an amount in the range of from 19 to 21 vol.-%.

Alternatively, by way of example, the gas stream provided in (ii) and contacted with the catalyst in (iii) may further contain 1 vol.-% or less of hydrogen, preferably 0.5 vol.-% or less, more preferably 0.1 vol.-% or less, more preferably 0.05 vol.-% or less, more preferably 0.001 vol.-% or less, more preferably 0.0005 vol.-% or less. It is, however, particularly preferred according to the present invention that the gas stream provided in (ii) and contacted with the catalyst in (iii) contains 0.0001 vol.-% or less of hydrogen.

According to the present invention, it is preferred that the gas stream provided in (ii) and contacted with the catalyst in (iii) further contains an inert gas in an amount in the range of from 5 to 90 vol.-%, preferably from 10 to 80 vol.-%, more preferably from 20 to 70 vol.-%, more preferably from 25 to 60 vol.-%, more preferably from 30 to 50 vol.-%, more preferably from 35 to 45 vol.-%, more preferably from 37 to 43 vol.-%. It is particularly preferred according to the present invention that the gas stream provided in (ii) and contacted with the catalyst in (iii) contains an inert gas in an amount in the range of from 39 to 41 vol.-%.

The type of inert gas which may be employed in the inventive process is not particularly restricted provided that under the chosen conditions it allows the conversion of 2-aminoethanol to ethane-1,2-diamine and/or linear poly-

ethylenimines of the formula $H_2N-[CH_2CH_2NH]_n-CH_2CH_2NH_2$ wherein $n \geq 1$. Thus, by way of example, the inert gas may comprise one or more gases selected from the group consisting of noble gases, $N_2$, and mixtures of two or more thereof, preferably from the group consisting of He, Ne, Ar, $N_2$, and mixtures of two or more thereof. It is, however, particularly preferred according to the present invention that the inert gas is Ar and/or $N_2$, preferably $N_2$.

According to the present invention, there is in principle no restriction as to the content of water in the gas stream provided in (ii) and contacted with the catalyst in (iii), provided that the content of $H_2O$ is of 5 vol.-% or less. Thus, by way of example, the gas stream provided in (ii) and contacted with the catalyst in (iii) may contain $H_2O$ in an amount of 3 vol.-% or less, preferably of 1 vol.-% or less, more preferably of 0.5 vol.-% or less, more preferably of 0.1 vol.-% or less, more preferably of 0.05 vol.-% or less, more preferably of 0.01 vol.-% or less, more preferably of 0.005 vol.-% or less, more preferably of 0.001 vol.-% or less, and more preferably of 0.0005 vol.-% or less. It is, however, particularly preferred according to the present invention that the gas stream provided in (ii) and contacted with the catalyst in (iii) contains 0.0001 vol.-% or less of water, preferably no water.

According to the present invention, the gas stream provided in (ii) is preferably heated prior to contacting with the catalyst in (iii). Thus, by way of example, the gas stream provided in (ii) may be heated to a temperature in the range of from 120 to 600°C, prior to contacting with the catalyst in (iii) at that temperature, preferably in the range of from 150 to 550°C, more preferably from 200 to 500°C, more preferably from 230 to 450°C, more preferably from 250 to 400°C, more preferably from 270 to 370°C, more preferably from 300 to 350°C, and more preferably from 320 to 340°C. It is, however, particularly preferred according to the present invention that the gas stream provided in (ii) is heated at a temperature of from 325 to 335°C prior to contacting with the catalyst in (iii) at that temperature.

According to the present invention, there is in principle no restriction as to the conditions for contacting the catalyst with the gas stream in (iii) provided that the conversion 2-aminoethanol to ethane-1,2-diamine and/or linear polyethylenimines takes place. Thus, by way of example, the contacting of the catalyst with the gas stream in (iii) may be effected at a pressure in the range of from 0.05 to 20 MPa, preferably from 0.1 to 10 MPa, more preferably from 0.3 to 5 MPa, more preferably from 0.5 to 3 MPa, more preferably from 0.6 to 2 MPa, more preferably from 0.7 to 1.5 MPa, more preferably from 0.8 to 1.3 MPa. It is, however, particularly preferred according to the present invention that the contacting of the catalyst with the gas stream in (iii) is effected in the range of from 0.9 to 1.1 MPa.

As regards the gas hourly space velocity (GHSV) for contacting of the catalyst with the gas stream in (iii), no particular restrictions apply such that in principle any conceivable gas hourly space velocity may be chosen for conducting the inventive process, provided that it is comprised in the range of from 100 to 30,000 h-1. Thus, by way of example, the contacting of the catalyst with the gas stream in (iii) may be effected at a gas hourly space velocity (GHSV) in the range of from 500 to 20,000 $h^{-1}$, preferably from 1,000 to 15,000 $h^{-1}$, more preferably from 2,000 to 10,000 $h^{-1}$, more preferably from 3,000 to 8,000 $h^{-1}$, more preferably from 4,000 to 6,000 $h^{-1}$, and more preferably from 4,500 to 5,500 $h^{-1}$. It is, however, particularly preferred that the catalyst with the gas stream in (iii) is effected at a gas hourly space velocity (GHSV) in the range of from 4,800 to 5,200 $h^{-1}$.

According to the present invention, there is in principle no restriction as to the $YO_2 : X_2O_3$ molar ratio of the zeolitic material such that in principle any conceivable $YO_2 : X_2O_3$ molar ratio may be chosen for conducting the inventive process. Thus, by way of example, the zeolitic material may display a $YO_2 : X_2O_3$ molar ratio in the range of from 5 to 100, preferably from 6 to 70, more preferably from 8 to 50, more preferably from 10 to 40, more preferably from 12 to 30, more preferably from 14 to 25, more preferably from 16 to 20. It is, however, particularly preferred that the zeolitic material displays a $YO_2 : X_2O_3$ molar ratio in the range of from 17 to 18.

As regards the tetravalent element Y of the zeolitic material having the MOR framework structure used in the inventive process, no particular restrictions apply such that in principle any conceivable tetravalent element may be chosen for conducting the inventive process. Thus, by way of example, Y may be selected from the group consisting of Si, Sn, Ti, Zr, Ge, and mixtures of two or more thereof. It is, however, particularly preferred according to the present invention that Y is Si.

As regards the trivalent element X of the zeolitic material having the MOR framework structure used in the inventive process, no particular restrictions apply such that in principle any conceivable trivalent element may be chosen for conducting the inventive process. Thus, by way of example, X may be selected from the group consisting of Al, B, In, Ga, and mixtures of two or more thereof. It is, however, particularly preferred according to the present invention that X is Al and/or B, preferably Al.

According to the present invention, it is preferred that the zeolitic material having the MOR framework structure is in the H-form and contains protons as extra-framework ions, wherein 0.1 wt.-% or less of the extra-framework ions are metal cations, calculated as the element and based on 100 wt.-% of $YO_2$ contained in the zeolitic material, preferably 0.05 wt.-% or less, more preferably 0.001 wt.-% or less, more preferably 0.0005 wt.-% or less. It is particularly preferred according to the present invention that the zeolitic material having the MOR framework structure is in the H-form and contains protons as extra-framework ions, wherein 0.0001 wt.-% or less of the extra-framework ions are metal cations, calculated as the element and based on 100 wt.-% of $YO_2$ contained in the zeolitic material.

Within the meaning of the present invention, "extra-framework ions" refer to ions and/or ionic compounds contained in the micropores of the zeolitic material and which compensate the charge of the zeolitic framework, wherein according to a preferred meaning of the present invention, "extra-framework ions" refer to cations and/or cationic compounds contained in the micropores of the zeolitic material and which compensate the charge of the zeolitic framework. According to the present invention, it is preferred that the zeolitic material having the MOR framework structure contains one or more metal ions M as extra-framework ions, wherein the one or more metal ions M are selected from the group consisting of alkaline earth metals and/or transition metals, more preferably from the group consisting of metals selected from group 4 and groups 6-11 of the Periodic Table of the Elements, preferably from group 4 and groups 8-11, wherein more preferably the one or more metal ions M are selected from the group consisting of Mg, Ti, Cu, Co, Cr, Ni, Fe, Mo, Mn, Ru, Rh, Pd, Ag, Os, Ir, Pt, Au, Sn, Zn, Ca, Mg and mixtures of two or more thereof, more preferably from the group consisting of Cu, Sn, Zn, Ca, Mg, and mixtures of two or more thereof. It is particularly preferred that the zeolitic material having the MOR framework structure contains Cu and/or Zn, preferably Cu as extra-framework ions.

As regards the content of M as extra-framework ions, no particular restrictions apply such that in principle any conceivable amount of M as extra-framework ions calculated as the element and based on 100 wt-% of $YO_2$ contained in the zeolitic material having the MOR framework structure may be chosen for conducting the inventive process. Thus, by way of example, the zeolitic material may contain from 0.5 to 15 wt.-% of M as extra-framework ions calculated as the element and based on 100 wt.-% of $YO_2$ contained in the zeolitic material having the MOR framework structure, preferably from 1 to 10 wt.-%, more preferably from 1.3. to 8 wt.-%, more preferably from 1.5 to 7 wt.-%, more preferably from 1.8 to 6 wt.-%, more preferably from 2 to 5.5 wt.-%, more preferably from 2.3 to 5 wt.-%, more preferably from 2.5 to 4.5 wt.-%, more preferably from 2.8 to 4 wt.-%, more preferably from 3 to 3.5 wt.-%. It is, however, particularly preferred according to the present invention that the zeolitic material contains from 3.1 to 3.4 wt.-% of M as extra-framework ions calculated as the element and based on 100 wt-% of $YO_2$ contained in the zeolitic material having the MOR framework structure.

Further, as regards the M : $X_2O_3$ molar ratio of the zeolitic material, no particular restrictions apply such that in principle any conceivable M : $X_2O_3$ molar ratio of the zeolitic material may be chosen for conducting the inventive process. Thus, by way of example, the M : $X_2O_3$ molar ratio of the zeolitic material may be in the range of from 0.01 to 2, preferably from 0.05 to 1.5, more preferably from 0.1 to 1, more preferably from 0.2 to 0.8, more preferably from 0.3 to 0.7, more preferably from 0.35 to 0.65, and more preferably from 0.4 to 0.6. It is, however, particularly preferred according to the present invention that the M : $X_2O_3$ molar ratio of the zeolitic material is in the range of from 0.45 to 0.55.

According to the present invention, it is preferred that the zeolitic material contains substantially no Na, preferably substantially no Na or K, more preferably substantially no alkali metal, and more preferably substantially no alkali metal or alkaline earth metals.

Within the meaning of the present invention, "substantially" as employed in the present invention with respect to the amount of Na, K, alkali metals or alkaline earth metals contained in the framework of the zeolitic material indicates an amount of 0.1 wt.-% or less of Na, K, alkali metals or alkaline earth metals calculated as the element and based on 100 wt.-% of $YO_2$ contained in the zeolitic material having the MOR framework structure, preferably 0.05 wt.-% or less, more preferably 0.001 wt.-% or less, more preferably 0.0005 wt.-% or less, and even more preferably 0.0001 wt.-% or less thereof.

It is preferred according to the present invention that the zeolitic material having the MOR framework structure comprises one or more zeolites selected from the group consisting of Mordenite, UZM-14, [Ga-Si-O]-MOR, Ca-Q, LZ-211, Maricopaite, Na-D, RMA-1, and mixtures of two or more thereof. It is particularly preferred according to the present invention that the zeolitic material having the MOR framework structure is UZM-14 and/or Mordenite, preferably Mordenite.

According to the present invention, it is preferred that the gas stream obtained in (iii) after contacting of the gas stream provided in (ii) with the catalyst provided in (i) displays an (ethane-1,2-diamine + diethylenetriamine) : (aminoethylethanolamine + piperazine) molar ratio of the total molar amount of ethane-1,2-diamine and diethylenetriamine to the total molar amount of aminoethylethanolamine and piperazine of more than 5, preferably of 5 to 80, more preferably of 5.5 to 50, more preferably of 6 to 30, more preferably of 6.5 to 20, more preferably of 7 to 15, more preferably of 7.5 to 12, more preferably of 8 to 11, more preferably of 8.5 to 10.5. It is particularly preferred according to the present invention that the gas stream obtained in (iii) after contacting of the gas stream provided in (ii) with the catalyst provided in (i) displays an (ethane-1,2-diamine + diethylenetriamine) : (aminoethylethanolamine + piperazine) molar ratio of the total molar amount of ethane-1,2-diamine and diethylenetriamine to the total molar amount of aminoethylethanolamine and piperazine of 9 to 10.

According to the present invention, it is preferred that at no point prior to the contacting in (iii) of the catalyst provided in (i) with the gas stream provided in (ii) has the zeolitic material having the MOR framework structure been subject to a treatment for the removal of $X_2O_3$ from its framework structure, and preferably to a treatment for the removal of $X_2O_3$ from the zeolitic material.

Within the meaning of the present invention, wherein preferably at no point prior to the contacting in (iii) of the catalyst provided in (i) with the gas stream provided in (ii) has the zeolitic material having the MOR framework structure been subjected to a treatment for the removal of $X_2O_3$ from its framework structure, this indicates that at no point has the zeolitic material been subject to a treatment wherein 5 mole-% or more of $X_2O_3$ based on 100 mole-% of $X_2O_3$ contained in the zeolitic material as synthesized has been removed from the framework structure of the zeolitic material, preferably 3 mole-% or more, more preferably 1 mole-% or more, more preferably 0.5 mole-% or more, more preferably 0.1 mole-% or more, more preferably 0.05 mole-% or more, more preferably 0.01 mole-% or more, more preferably 0.005 mole-% or more, more preferably 0.001 mole-% or more, more preferably 0.0005 mole-% or more, and more preferably 0.0001 mole-% or more.

According to the meaning of the present invention wherein it is preferred that at no point prior to the contacting in (iii) of the catalyst provided in (i) with the gas stream provided in (ii) has the zeolitic material having the MOR framework structure been subjected to a treatment for the removal of $X_2O_3$ from the zeolitic material, this indicates that at no point has the zeolitic material been subject to a treatment wherein 5 mole-% or more of $X_2O_3$ based on 100 mole-% of $X_2O_3$ contained in the zeolitic material as synthesized has been removed from the zeolitic material, preferably 3 mole-% or more, more preferably 1 mole-% or more, more preferably 0.5 mole-% or more, more preferably 0.1 mole-% or more, more preferably 0.05 mole-% or more, more preferably 0.01 mole-% or more, more preferably 0.005 mole-% or more, more preferably 0.001 mole-% or more, more preferably 0.0005 mole-% or more, and more preferably 0.0001 mole-% or more.

As regards the preparation of the zeolitic material having the MOR framework structure used in the inventive process, no particular restrictions apply such that in principle any conceivable zeolitic material having the MOR framework structure may be chosen for conducting the inventive process. It is, however, preferred according to the present invention that the zeolitic material having the MOR framework structure is prepared by a process comprising

(1) preparing a mixture comprising at least one source of $YO_2$, at least one source of $X_2O_3$, and comprising one or more organotemplates as structure directing agent and/or comprising seed crystals;
(2) crystallizing the mixture prepared in (i) for obtaining a zeolitic material having the MOR framework structure;
(3) optionally isolating the zeolitic material obtained in (2);
(4) optionally washing the zeolitic material obtained in (2) or (3);
(5) optionally drying and/or calcining the zeolitic material obtained in (2), (3), or (4);
(6) optionally subjecting the zeolitic material obtained in (2), (3), (4), or (5) to an ion-exchange procedure, wherein extra-framework ions contained in the zeolitic material are ion-exchanged against $H^+$;
(7) optionally subjecting the zeolitic material obtained in (2), (3), (4), (5), or (6) to an ion-exchange procedure, wherein extra-framework ions contained in the zeolitic material are ion-exchanged against one or more metal ions M selected from the group consisting of alkaline earth metals and/or transition metals, more preferably from the group consisting of metals selected from group 4 and groups 6-11 of the Periodic Table of the Elements, preferably from group 4 and groups 8-11, wherein more preferably the one or more metal ions M are selected from the group consisting of Mg, Ti, Cu, Co, Cr, Ni, Fe, Mo, Mn, Ru, Rh, Pd, Ag, Os, Ir, Pt, Au, Sn, Zn, Ca, Mg and mixtures of two or more thereof, more preferably from the group consisting of Cu, Sn, Zn, Ca, Mg, and mixtures of two or more thereof, wherein more preferably the extra-framework ions contained in the zeolitic material are ion-exchanged against Cu and/or Zn, preferably Cu;
(8) optionally drying and/or calcining the zeolitic material obtained in (7).

Within the meaning of the present invention, the term "organotemplate" as employed in the present application designates any conceivable organic material which is suitable for template-mediated synthesis of a zeolite material, preferably of a zeolite material having a MOR-type framework-structure, and even more preferably which is suitable for the synthesis of UZM-14 and/or Mordenite.

It is preferred according to the present invention that the one or more organotemplates comprised in the mixture prepared in (1) is selected from the group consisting of tetraalkylammonium containing compounds and tetraalkylphosphonium containing compounds, preferably from the group consisting of tetraalkylammonium cation $R^1R^2R^3R^4N^+$-containing compounds and tetraalkylphosphonium cation $R^1R^2R^3R^4P^+$-containing compounds, wherein $R^1$, $R^2$, $R^3$, and $R^4$ independently from one another stand for optionally substituted and/or optionally branched $(C_1-C_6)$alkyl, preferably $(C_1-C_5)$alkyl, more preferably $(C_1-C_4)$alkyl, more preferably $(C_1-C_3)$alkyl, and even more preferably for optionally substituted methyl or ethyl, wherein even more preferably $R^1$, $R^2$, $R^3$, and $R^4$ stand for optionally substituted ethyl, preferably for unsubstituted ethyl.

It is further preferred that the one or more tetraalkylammonium cation $R^1R^2R^3R^4N^+$-containing compounds and/or that the one or more tetraalkylphosphonium cation $R^1R^2R^3R^4P^+$-containing compounds are salts, preferably one or more salts selected from the group consisting of halides, preferably chloride and/or bromide, more preferably chloride, hydroxide, sulfate, nitrate, phosphate, acetate, and mixtures of two or more thereof, more preferably from the group consisting of chloride, hydroxide, sulfate, and mixtures of two or more thereof, more preferably the one or more tetraalkylammonium cation $R^1R^2R^3R^4N^+$-containing compounds and/or the one or more tetraalkylphosphonium cation

$R^1R^2R^3R^4P^+$-containing compounds are hydroxides and/or bromides, and even more preferably bromides.

It is preferred according to the present invention that the one or more organotemplates comprised in the mixture prepared in (1) is selected from the group consisting of $N,N,N,N$-tetra($C_1$-$C_4$)alkylammonium and $N,N,N,N$-tetra($C_1$-$C_4$)alkylphosphonium compounds, preferably from the group consisting of $N,N,N,N$-tetra($C_1$-$C_3$)alkylammonium and $N,N,N,N$-tetra($C_1$-$C_3$)alkylphosphonium compounds, more preferably from the group consisting of $N,N,N,N$-tetra($C_1$-$C_2$)alkylammonium and $N,N,N,N$-tetra($C_1$-$C_2$)alkylphosphonium compounds, more preferably from the group consisting of $N,N,N,N$-tetra($C_1$-$C_2$)alkylammonium and $N,N,N,N$-tetra($C_1$-$C_2$)alkylphosphonium compounds, more preferably from the group consisting of $N,N,N,N$-tetraethylammonium compounds, $N,N,N,N$-tetramethylammonium compounds, $N,N,N,N$-tetraethylphosphonium compounds, $N,N,N,N$-tetramethylphosphonium compounds, and mixtures of two or more thereof. It is particularly preferred according to the present invention that the one or more organotemplates comprise one or more $N,N,N,N$-tetraethylammonium or $N,N,N,N$-tetraethylphosphonium compounds, preferably one or more $N,N,N,N$ tetraethylammonium compounds.

As regards the organotemplate : $YO_2$ molar ratio of the one or more organotemplates to $YO_2$ in the mixture provided according to (1) for preparing the zeolitic material having the MOR framework structure, no particular restrictions apply such that in principle any conceivable organotemplate : $YO_2$ molar ratio may be chosen for conducting the process for preparing the zeolitic material having the MOR framework structure. Thus, by way of example, the organotemplate : $YO_2$ molar ratio of the one or more organotemplates to $YO_2$ in the mixture provided according to (1) may range from 0.005 to 0.14, preferably from 0.01 to 0.3, more preferably from 0.02 to 0.2, more preferably from 0.025 to 0.14, more preferably from 0.03 to 0.1, more preferably from 0.035 to 0.08, more preferably from 0.04 to 0.06. It is, however, particularly preferred according to the present invention that the organotemplate : $YO_2$ molar ratio of the one or more organotemplates to $YO_2$ in the mixture provided according to (1) ranges from 0.045 to 0.055.

It is alternatively preferred according to the present invention that the mixture prepared in (1) and crystallized in (2) contains substantially no organotemplates with the exception of organotemplate which may optionally be contained in the micropores of the zeolitic material preferably employed as seed crystals, wherein more preferably the mixture prepared in (1) and crystallized in (2) contains substantially no organotemplates. It is, however, preferred according to the present invention that the mixture prepared in (1) comprises one or more organotemplates as structure directing agent.

Within the meaning of the present invention wherein the mixture prepared in (1) and crystallized in (2) contains substantially no organotemplates, this indicates that the mixture prepared in (1) and crystallized in (2) may only contain organotemplates in an amount of 0.1 wt.-% or less based on 100 wt.-% of $YO_2$ contained in the mixture, and preferably in an amount of 0.05 wt.-% or less, more preferably of 0.001 wt.-% or less, more preferably of 0.0005 wt.-% or less, and even more preferably in an amount of 0.0001 wt.-% or less based on 100 wt.-% of $YO_2$ contained in the mixture. Said amounts of organotemplates, if at all present in the mixture prepared in (1) and crystallized in (2), may also be denoted as "impurities" or "trace amounts" within the meaning of the present invention. Furthermore, it is noted that the terms "organotemplate" and "organic structure directing agent" are synonymously used in the present application.

It is preferred according to the present invention that the mixture prepared in (1) and crystallized in (2) contains substantially no zeolitic material, wherein preferably the mixture prepared in (1) and crystallized in (2) contains substantially no seed crystals.

Within the meaning of the present invention wherein the mixture prepared in (1) and crystallized in (2) contains substantially no zeolitic material and preferably contains substantially no seed crystals, this indicates that the mixture prepared in (1) and crystallized in (2) may only contain zeolitic material and preferably may only contain seed crystals in an amount of 0.1 wt.-% or less based on 100 wt.-% of $YO_2$ contained in the mixture, and preferably in an amount of 0.05 wt.-% or less, more preferably of 0.001 wt.-% or less, more preferably of 0.0005 wt.-% or less, and even more preferably in an amount of 0.0001 wt.-% or less based on 100 wt.-% of $YO_2$ contained in the mixture. Said amounts of zeolitic material and preferably of seed crystals, if at all present in the mixture prepared in (1) and crystallized in (2), may also be denoted as "impurities" or "trace amounts" within the meaning of the present invention.

It is preferred that in (6) of the process for preparing the zeolitic material having the MOR framework structure used in the inventive process the step of subjecting the zeolitic material to an ion-exchange procedure includes the steps of

(6.a) subjecting the zeolitic material obtained in (2), (3), (4), or (5) to an ion-exchange procedure, wherein extra-framework ions contained in the zeolitic material are ion-exchanged against $NH_4^+$;
(6.b) calcining the ion-exchanged zeolitic material obtained in (6.a) for obtaining the H-form of the zeolitic material.

As regards calcining in (5), (6.b), (8) and/or (12), no particular restrictions apply such that in principle any conceivable temperature and/or duration may be chosen for conducting the process for preparing the zeolitic material having the MOR framework structure.

Thus, by way of example, calcining in (5), (6.b), (8) and/or (12) may be conducted at a temperature in the range of from 200 to 850°C, preferably of from 250 to 800°C, more preferably of from 300 to 750°C, more preferably of from 350 to 700°C, more preferably of from 400 to 650°C, more preferably of from 450 to 620°C, more preferably of from 500 to 600°C, and more preferably of from 520 to 580°C. It is, however, particularly preferred according to the present invention that calcining in (5), (6.b), (8) and/or (12) is conducted at a temperature in the range of from 540 to 560°C.

Further, by way of example, calcining of the zeolitic material in (5), (6.b), (8) and/or (12) may be effected in batch mode, in semi-continuous mode, or in continuous mode. Calcination in (6.b) is performed by heating of the zeolitic material to a temperature according to any of the particular and performed embodiments defined in the present application and holding it at that temperature for a duration ranging from 0.5 to 36 h, preferably from 1 to 32 h, more preferably from 2 to 28 h, more preferably from 4 to 24 h, more preferably from 6 to 20 h, more preferably from 8 to 18 h, and more preferably from 10 to 14 h. It is, however, particularly preferred according to the present invention that calcining of the zeolitic material in (5), (6.b), (8) and/or (12) is effected by heating of the zeolitic material to a given temperature and holding it at that temperature for a duration ranging from 11.5 to 12.5 h. Furthermore, it is particularly preferred according to the present invention that calcining in (5), (6.b), (8) and/or (12) is conducted at a temperature in the range of from 540 to 560°C for a duration ranging from 11.5 to 12.5 h. When conducted in semi-continuous or in continuous mode, the duration of calcination corresponds to the residence time of the zeolitic material in the given calciner operating in a semi-continuous mode or in continuous mode.

In case the process is carried out in a larger scale, it is preferred to perform the calcination in semi-continuous mode or in continuous mode, more preferably in continous mode. Even more preferably, calcining the zeolitic material in (5), (6.b), (8) and/or (12) is carried out in continuous mode with a rate in the range of from 0.2 to 50.0 kg of the zeolitic material per hour, preferably from 0.5 to 2.0 kg of the zeolitic material per hour. Conceivable apparatuses which can be used for such a preferred continuous calcination include, for example, a band calciner and/or a rotary calciner, wherein preferably a rotary calciner is used.

According to the present invention, it is however particularly preferred that, if the zeolitic material obtained in (7) which is ion-exchanged with one or more metal ions M is subject to a heating treatment such as drying and/or calcination, said treatment does not involve a temperature of 540°C or greater, and preferably does not involve a temperature of 520°C or greater, more preferably of 500°C or greater, more preferably of 450°C or greater, more preferably of 400°C or greater, more preferably of 350°C or greater, more preferably of 300°C or greater, more preferably of 250°C or greater, and more preferably of 200°C. According to the present invention it is particularly preferred that zeolitic material obtained in (7) which is ion-exchanged with one or more metal ions M is not subject to a temperature of 150°C or greater. Thus, according to said particularly preferred embodiments, the zeolitic material obtained in (7) which is ion-exchanged with one or more metal ions M is not subject to calcination according to (8) as defined in any of the particular and preferred embodiments of the present application.

It is preferred according to the present invention that in (7) the zeolitic material is ion-exchanged such as to obtain a loading of the one or more metal ions M in the zeolitic material ranging from 0.1 to 10 wt.-% calculated as the one or more elements M and based on 100 wt.-% of $YO_2$ contained in the zeolitic material, preferably from 0.5 to 8 wt.-%, more preferably from 1 to 6 wt.-%, more preferably from 1.2 to 5 wt.-%, more preferably from 1.5 to 4 wt.-%, more preferably from 1.8 to 3.5 wt.-%, more preferably from 2 to 3 wt.-%, more preferably from 2.3 to 2.9 wt.-%. It is particularly preferred according to the present invention that in (7) the zeolitic material is ion-exchanged such as to obtain a loading of the one or more metal ions M in the zeolitic material ranging from 2.5 to 2.7 wt.-%.

As regards the element Y used for preparing the zeolitic material having the MOR framework structure, no particular restrictions apply such that in principle any conceivable tetravalent element may be chosen for conducting the process for preparing the zeolitic material having the MOR framework structure. Thus, by way of example, Y may be selected from the group consisting of Si, Sn, Ti, Zr, Ge, and combinations of two or more thereof. It is, however, particularly preferred according to present invention that Y is Si.

It is preferred according to the present invention that the at least one source for $YO_2$ comprises one or more compounds selected from the group consisting of silicas, silicates, and mixtures thereof, preferably from the group consisting of fumed silica, silica hydrosols, reactive amorphous solid silicas, silica gel, silicic acid, water glass, sodium metasilicate hydrate, sesquisilicate, disilicate, colloidal silica, silicic acid esters, tetraalkoxysilanes, and mixtures of two or more thereof, more preferably from the group consisting of fumed silica, silica hydrosols, silica gel, silicic acid, water glass, colloidal silica, silicic acid esters, tetraalkoxysilanes, and mixtures of two or more thereof, more preferably from the group consisting of fumed silica, silica hydrosols, silica gel, colloidal silica, and mixtures of two or more thereof, more preferably from the group consisting of fumed silica, silica gel, colloidal silica, and mixtures of two or more thereof, more preferably the at least one source of $YO_2$ is selected from the group consisting of fumed silica, colloidal silica, and mixtures thereof. It is particularly preferred according to the present invention that fumed silica is employed as the source of $YO_2$.

As regards the element X used for preparing the zeolitic material having the MOR framework structure, no particular restrictions apply such that in principle any conceivable trivalent element may be chosen for conducting the process for preparing the zeolitic material having the MOR framework structure. Thus, by way of example, X may be selected from the group consisting of Al, B, In, Ga, and combinations of two or more thereof. It is, however, particularly preferred according to the present invention that X is Al.

It is preferred according to the present invention the at least one source for $X_2O_3$ comprises one or more aluminum salts, preferably an aluminate of an alkali metal, wherein the alkali metal is preferably selected from the group consisting of Li, Na, K, Rb, and Cs, wherein more preferably the alkali metal is Na and/or K, and wherein even more preferably the alkali metal is Na.

As regards the $YO_2 : X_2O_3$ molar ratio of the mixture prepared in (1), no particular restrictions apply such that in principle any conceivable $YO_2 : X_2O_3$ molar ratio may be chosen for conducting the process for preparing the zeolitic material having the MOR framework structure used in the inventive process. Thus, by way of example, the $YO_2 : X_2O_3$ molar ratio of the mixture prepared in (1) may range from 2 to 50, preferably from 4 to 40, more preferably from 6 to 35, more preferably from 10 to 30, more preferably from 13 to 25, more preferably from 15 to 23, more preferably from 17 to 22. It is particularly preferred according to the present invention that the $YO_2 : X_2O_3$ molar ratio of the mixture prepared in (1) ranges from 19 to 21.

As regards the seed crystals used for the process of preparing the zeolitic material having the MOR framework structure, no particular restrictions apply such that in principle any conceivable seed crystals may be chosen for preparing the zeolitic material having the MOR framework structure. Thus, by way of example, the seed crystals may comprise a zeolitic material, preferably one or more zeolites, more preferably one or more zeolites having a BEA framework structure, wherein more preferably the seed crystals comprise zeolite beta. It is, however, particularly preferred according to the present invention that zeolite beta is employed as the seed crystals for preparing the mixture in (1).

Same applies to the amount of seed crystals used for the process of preparing the zeolitic material having the MOR framework structure provided that the zeolitic material having the MOR framework structure can be prepared. Thus, by way of example, the amount of seed crystals in the mixture prepared in (1) may range from 0.1 to 15 wt.-% based on 100 wt.-% of $YO_2$ contained in the mixture, preferably from 0.5 to 10 wt.-%, more preferably from 0.8 to 8 wt.-%, more preferably from 1 to 5 wt.-%, more preferably from 1.3 to 3 wt.-%. It is, however, particularly preferred according to the present invention that the amount of seed crystals in the mixture prepared in (1) ranges from 1.5 to 2.5 wt.-%.

According to the present invention, it is preferred that the mixture prepared in (1) further comprises a solvent system containing one or more solvents, wherein the solvent system preferably comprises one or more solvents selected from the group consisting of polar protic solvents and mixtures thereof, preferably from the group consisting of n-butanol, isopropanol, propanol, ethanol, methanol, water, and mixtures thereof, more preferably from the group consisting of ethanol, methanol, water, and mixtures thereof, wherein more preferably the solvent system comprises water. It is particularly preferred according to the present invention that water, preferably deionized water, is used as the solvent system.

According to the present invention, it is further preferred that when the mixture prepared in (1) and crystallized in (2) comprises one or more organotemplates, and when the mixture prepared in (1) comprises water as the solvent system, the $H_2O : YO_2$ molar ratio of the mixture prepared in (1) ranges from 5 to 70, preferably from 10 to 65, more preferably from 15 to 60, more preferably from 20 to 55, more preferably from 25 to 50, more preferably from 30 to 47, more preferably from 35 to 45, more preferably from 37 to 43. It is particularly preferred that when the mixture prepared in (1) and crystallized in (2) comprises one or more organotemplates, and when the mixture prepared in (1) comprises water as the solvent system, the $H_2O : YO_2$ molar ratio of the mixture prepared in (1) ranges from 39 to 41.

It is preferred according to the present invention that when the mixture prepared in (1) and crystallized in (2) comprises seed crystals, and when the mixture prepared in (1) comprises water as the solvent system, the $H_2O : YO_2$ molar ratio of the mixture prepared in (1) ranges from 5 to 45, preferably from 10 to 40, more preferably from 12 to 35, more preferably from 15 to 30, more preferably from 17 to 27, more preferably from 19 to 25. It is particularly preferred that when the mixture prepared in (1) and crystallized in (2) comprises seed crystals, and when the mixture prepared in (1) comprises water as the solvent system, the $H_2O : YO_2$ molar ratio of the mixture prepared in (1) ranges from 21 to 23.

According to the present invention, it is preferred that the mixture prepared in (1) further comprises one or more alkali metals (AM), preferably the one or more alkali metals are selected from the group consisting of Li, Na, K, Cs, and mixtures thereof, more preferably the mixture prepared in (1) further comprises Na and/or K, preferably Na as the alkali metal AM.

As regards the $AM : YO_2$ molar ratio of alkali metals to $YO_2$ in the mixture prepared in (1) when the mixture prepared in (1) and crystallized in (2) comprises one or more organotemplates, no particular restrictions apply such that any conceivable $AM : YO_2$ molar ratio may be chosen for the process of preparing the zeolitic material having the MOR framework structure used in the inventive process. Thus, by way of example, when the mixture prepared in (1) and crystallized in (2) comprises one or more organotemplates, the $AM : YO_2$ molar ratio of alkali metals to $YO_2$ in the mixture prepared in (1) may range from 0.01 to 1.5, preferably from 0.05 to 1, more preferably from 0.08 to 0.5, more preferably from 0.1 to 0.35, more preferably from 0.12 to 0.3, more preferably from 0.15 to 0.25. It is, however, particularly preferred according to the present invention that the $AM : YO_2$ molar ratio of alkali metals to $YO_2$ in the mixture prepared in (1), when the mixture prepared in (1) and crystallized in (2) comprises one or more organotemplates, ranges from 0.18 to 0.22.

As regards the $AM : YO_2$ molar ratio of alkali metals to $YO_2$ in the mixture prepared in (1) when the mixture prepared in (1) and crystallized in (2) comprises seed crystals, no particular restrictions apply such that any conceivable

AM : YO$_2$ molar ratio may be chosen for the process of preparing the zeolitic material having the MOR framework structure used in the inventive process. Thus, by way of example, when the mixture prepared in (1) and crystallized in (2) comprises seed crystals, the AM : YO$_2$ molar ratio of alkali metals to YO$_2$ in the mixture prepared in (1) may range from 0.3 to 2, preferably from 0.5 to 1.5, more preferably from 0.8 to 1.2, more preferably from 1 to 1, more preferably from 1.2 to 0.8, more preferably from 1.3 to 0.5. It is, however, particularly preferred according to the present invention that the AM : YO$_2$ molar ratio of alkali metals to YO$_2$ in the mixture prepared in (1), when the mixture prepared in (1) and crystallized in (2) comprises seed crystals, ranges from 1.35 to 1.4.

According to the present invention, it is further preferred that the YO$_2$: X$_2$O$_3$ : AM molar ratio of the mixture prepared in (1) ranges from 1 : (0.02-0.5) : (0.1-2), preferably from 1 : (0.025-0.25) : (0.2-1.5), more preferably from 1 : (0.029-0.17) : (0.3-1.4), more preferably from 1 : (0.033-0.1) : (0.4-1.2), more preferably from 1 : (0.04-0.08) : (0.5-1), more preferably from 1 : (0.043-0.7) : (0.55-0.9), more preferably from 1 : (0.045-0.06) : (0.6-0.8), and more preferably from 1 : (0.045-0.05) : (0.65-0.75).

As regards the crystallization in (2), no particular restrictions apply such that in principle any conceivable conditions of crystallization may be chosen for the process of preparing the zeolitic material having the MOR framework structure.

Thus, by way of example, the crystallization in (2) may involve heating of the mixture prepared in (1), preferably to a temperature ranging from 75 to 210°C, more preferably from 90 to 200°C, more preferably from 110 to 190°C, more preferably from 130 to 175°C, more preferably from 140 to 165°C. It is, however, particularly preferred according to the present invention that the crystallization in (2) involves heating of the mixture prepared in (1) to a temperature ranging from 145 to 155°C.

Further, by way of example, the crystallization in (2) may be conducted under autogenous pressure, preferably under solvothermal conditions. It is, however, particularly preferred according to the present invention that the crystallization in (2) is conducted under hydrothermal conditions.

According to the present invention, it is preferred that when the mixture prepared in (1) and crystallized in (2) comprises one or more organotemplates, the crystallization in (2) involves heating of the mixture prepared in (1) for a period in the range of from 50 to 115 h, more preferably from 60 to 95 h, more preferably from 65 to 85 h, more preferably from 70 to 80 h, more preferably from 70 to 78 h, and more preferably from 75 to 77 h.

According to the present invention, it is preferred that when the mixture prepared in (1) and crystallized in (2) comprises seed crystals, the crystallization in (2) involves heating of the mixture prepared in (1) for a period in the range of from 60 to 140 h, more preferably from 70 to 120 h, more preferably from 75 to 100 h, more preferably from 80 to 90 h, and more preferably from 82 to 86 h.

It is preferred according to the present invention that the zeolitic material having an MOR framework structure crystallized in (2) is Mordenite.

It is preferred according to the present invention that the mixture prepared in (1) and crystallized in (2) contains substantially no phosphorous.

Within the meaning of the present invention, "substantially" as employed in the present invention with respect to the amount of phosphorous contained in the mixture prepared in (1) and crystallized in (2) indicates an amount of 0.1 wt.-% or less of phosphorous calculated as the element and based on 100 wt.-% of YO$_2$ in the mixture, preferably 0.05 wt.-% or less, more preferably 0.001 wt.-% or less, more preferably 0.0005 wt.-% or less, and even more preferably 0.0001 wt.-% or less thereof. Within the meaning of the present invention, the definition of phosphorous substantially not being contained in the mixture prepared in (1) and crystallized in (2) comprises both elemental phosphorous as well as phosphorous containing compounds. According to the present invention, it is preferred that the framework of the zeolitic material obtained in (2) contains substantially no phosphorous, more preferably the zeolitic material obtained in (2) contains substantially no phosphorous.

Within the meaning of the present invention, "substantially" as employed in the present invention with respect to the amount of phosphorous contained in the framework of the zeolitic material obtained in (2) indicates an amount of 0.1 wt.-% or less of phosphorous calculated as the element and based on 100 wt.-% of YO$_2$ in the zeolitic material, preferably 0.05 wt.-% or less, more preferably 0.001 wt.-% or less, more preferably 0.0005 wt.-% or less, and even more preferably 0.0001 wt.-% or less thereof. Furthermore, within the meaning of the present invention, "substantially" as employed in the present invention with respect to the amount of phosphorous contained in the zeolitic material obtained in (2) indicates an amount of 0.1 wt.-% or less of phosphorous calculated as the element and based on 100 wt.-% of YO$_2$ in the zeolitic material, and preferably 0.05 wt.-% or less, more preferably 0.001 wt.-% or less, more preferably 0.0005 wt.-% or less, and even more preferably 0.0001 wt.-% or less thereof. Within the meaning of the present invention, the definition of phosphorous substantially not being contained in the framework of the zeolitic material obtained in (2) and preferably not being contained in the mixture prepared in (1) and crystallized in (2) comprises both elemental phosphorous as well as phosphorous containing compounds.

There is no particular restriction according to the present invention as to the form in which the zeolitic material having the MOR framework structure may be provided in the catalyst employed in the inventive process. Thus, the zeolitic material may be used as such, or may be employed together with further components. According to the inventive process it is thus preferred that the zeolitic material is comprised in the catalyst employed in the inventive process in the form of a molding. Accordingly it is preferred according to the present invention that the preferred process for preparing the zeolitic material according to any of the particular preferred embodiments described in the present application further comprises

(9) mixing the zeolitic material obtained in (2), (3), (4), (5), (6), (7) or (8) with one or more binders;
(10) kneading of the mixture obtained in (9);
(11) molding of the kneaded mixture obtained in (10) to obtain one or more moldings; and
(12) drying and/or calcining the one or more moldings obtained in (11).

With respect to the one or more binders with which the zeolitic material obtained in (7) or (8) may be mixed, no particular restrictions apply such that in principle any suitable binder may be employed. Thus, by way of example, the one or more binders may be selected from the group consisting of inorganic binders, wherein according to the present invention it is preferred that the one or more binders comprise one or more sources of a metal oxide and/or of a metalloid oxide or one or more sources of graphite, wherein the one or more sources of a metal oxide and/or of a metalloid oxide are preferably selected from the group consisting of silica, alumina, titania, zirconia, lanthana, magnesia, and mixtures and/or mixed oxides of two or more thereof, more preferably from the group consisting of silica, alumina, titania, zirconia, magnesia, silica-alumina mixed oxides, silica-titania mixed oxides, silica-zirconia mixed oxides, silica-lanthana mixed oxides, silica-zirconia-lanthana mixed oxides, alumina-titania mixed oxides, alumina-zirconia mixed oxides, alumina-lanthana mixed oxides, alumina-zirconia-lanthana mixed oxides, titania-zirconia mixed oxides, and mixtures and/or mixed oxides of two or more thereof, more preferably from the group consisting of silica, alumina, silica-alumina mixed oxides, and mixtures of two or more thereof. According to the present invention it is however particularly preferred that the one or more binders comprise one or more sources of silica, alumina, zirconia, and/or graphite, wherein more preferably the binder consists of one or more sources of silica, alumina, zirconia, and/or graphite, wherein more preferably the one or more binders comprise one or more sources of silica, alumina, and/or zirconia, wherein even more preferably the binder consists of one or more sources silica, alumina, and/or zirconia, preferably of silica, alumina, and/or zirconia.

According to the present invention, it is preferred that 2-aminoethanol comprised in the gas stream obtained in (iii) is separated from said gas stream and recycled to (ii).

DESCRIPTION OF THE FIGURES

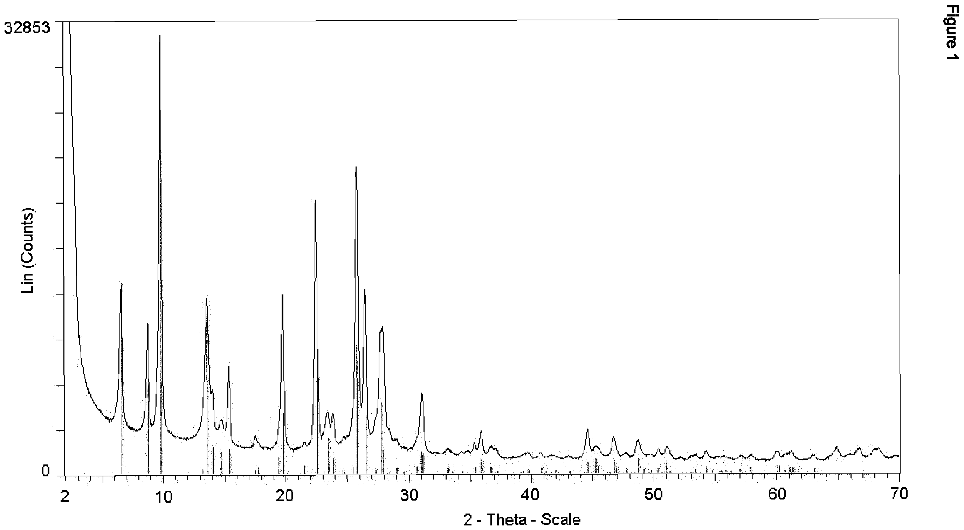

Figure 1   shows the powder X-ray diffraction pattern of the UZM-14-B according to US 7,687,423 B2 obtained in Example 3, wherein the line pattern of sodium Mordenite from a crystallographic database has been included for comparative purposes. The X-ray diffraction pattern shown in the figure was measured using Cu K alpha-1 radiation. In the respective diffractogram, the diffraction angle 2 theta in ° is shown along the abscissa and the intensities are plotted along the ordinate.

EXAMPLES

The crystallite size of the samples was determined using X-ray diffraction by fitting the diffracted peak width using the software TOPAS 4.2. Instrumental broadening was considered during the peak fitting using the fundamental parameter approach as described in TOPAS 4.2 Users Manual (Bruker AXS GmbH, Ostliche Rheinbrückenstr. 49, 76187 Karlsruhe, Germany). This leads to a separation of the instrumental from the sample broadening. The sample contribution was determined using a single Lorentzian profile function that is defined in the following equation:

$$\beta = \lambda / (L \cdot \cos\theta)$$

where $\beta$ is the Lorentzian full width at half maximum (FWHM), $\lambda$ is the X-ray wavelength of the CuKa radiation used, L is the crystallite size, and $\theta$ is the half the scattering angle of the peak position.

The crystallite size of the 002 reflection in samples having the MOR framework type was determined in a refinement of the local data surrounding the 002 reflection, from 21° to 24.2°(2θ). Single peaks with varying crystallite sizes model the surrounding reflections.

The data was collected in the Bragg-Brentano geometry from 2° to 70° (2θ), using a step size of 0.02° (2θ).

**Example 1:** Synthesis of H-Mordenite

In a 5 l plastic beaker 120 g fumed silica (CAB-O-SIL M5, Sigma-Aldrich) are suspended in 900 g deionized water. To this suspension a mixture of 52.04 g tetraethylammonium bromide (TEABr, Aldrich) in 161.7 g deionized water is added. The resulting mixture is agitated for 1 h at a stirring speed of 200 rpm. Then, a mixture of 36.5 g sodium hydroxide flakes (NaOH, Sigma-Aldrich) in 161.7 g deionized water is added. The resulting mixture is then agitated for 1.5 h at a stirring speed of 300 rpm. Subsequently, 188.6 g deionized water are added and then a mixture of 15.66 g sodium aluminate (NaAlO$_2$, Sigma-Aldrich) in 188.6 g deionized water. The resulting mixture is then agitated for 1 h at a stirring speed of 200 rpm. The pH value of the mixture was determined to be 12.2. A gel is formed which aged over night.

The synthetic gel displaying a molar composition of 0.28 Na$_2$O : 0.048 Al$_2$O$_3$ : SiO$_2$ : 44.5 H$_2$O : 0.13 TEABr is then crystallized in a pressure tight vessel for 72 h at 170 °C under agitating at a stirring speed of 250 rpm. Then, the resulting product is filtered off as a solid and washed with deionized water until the electrical conductance of the washing water reaches a value lower than 150 µS. The solids are then dried in air at 90 °C for 12 h. Subsequently, the solids are heated in air to 90 °C with a heating rate of 3.5 °C per minute and then left at said temperature for 2 h. Then the solids are heated to 120 °C with a heating rate of 1.5 °C per minute and then left at said temperature for 2 h. Then the solids are heated to 550 °C with a heating rate of 4.5 °C/min and left at said temperature for 12 h. The yield was 82 g.

According to the elemental analysis the resulting product had the following contents determined per 100 g substance of < 0.1 g carbon, 4.9 g aluminum, 3.2 g sodium and 37 g silicon.

The BET surface area was determined to be 404 m$^2$/g. The crystallinity of the product was measured to be 90%.

As taken from the X-ray diffraction pattern of the resulting product, the zeolitic material obtained displays the MOR framework structure as the single crystalline phase, wherein the average crystal size as calculated from calculated from the X-ray diffraction data was determined to be 59 nm, and the average crystal size along the 002 axis of the crystallites was determined to be 46 nm.

In a 2 liter stirring apparatus, 70 g of ammonium nitrate were placed as an aqueous solution (10 wt.-% NH$_4$NO$_3$), 70 g of the zeolitic material were added, and the resulting mixture was stirred for 2 h at 80°C. The zeolitic material was then filtered off and washed with 630 g of distilled water. The filtrate was discarded and a new 10-wt.% aqueous solution containing 70 g of ammonium nitrate was then placed in the stirring apparatus to which the washed zeolitic material was added and the resulting mixture again stirred for 2h at 80°C. The zeolitic material was then filtered off and washed anew with 630 g of distilled water. The washed material was then dried for 5 h at 120°C and subsequently calcined at 500°C for 5 h with a heating rate of 2°C/min. The entire procedure was then repeated, affording 63.4 g of the H-form of the zeolitic material.

According to the elemental analysis, the resulting sample had the following contents determined per 100 g substance of <0.1 g carbon, 5.0 g aluminum, 0.01 g sodium and 38 g silicon.

The BET surface area was determined to be 474 m$^2$/g.

**Example 2:** Synthesis of copper-exchanged Mordenite

1.5 liters of a 0.01 molar aqueous solution of copper(II) acetate (3 grams in 1.5 liters) were placed in a 2 liter stirring apparatus and 25 g of the product from Example 1 were then added and the mixture stirred at room temperature for 20 h. The zeolitic material was then filtered off, and the filtrate was discarded. A new solution of 1.5 liters of a 0.01 molar aqueous solution of copper(II) acetate (3 grams in 1.5 liters) was then placed in the 2 liter stirring apparatus and the zeolitic material was added thereto and the mixture stirred at room temperature for 20 h. The zeolitic material was then filtered off, the filtrate discarded, and the zeolitic material was again added to a new solution of 1.5 liters of a 0.01 molar aqueous solution of copper(II) acetate (3 grams in 1.5 liters) and stirred for 20 h at room temperature. The resulting product was then separated from the solution by centrifugation, the solution discarded, and the zeolitic material subsequently suspended in 1.25 liters of distilled water. The zeolitic material was then separated from the solution by centrifugation, the washwater was discarded, and the washing procedure with distilled water was repeated 3 times for washing the zeolitic material. The zeolitic material was then dried for 24 h at 110°C, thus affording 24.4 g of a copper-exchanged zeolitic material.

According to the elemental analysis the resulting product had the following contents determined per 100 g substance of < 0.1 g carbon, 4.8 g aluminum, 2.6 g copper and 35 g silicon.

The BET surface area was determined to be 371 m$^2$/g.

**Example 3:** Synthesis of UZM-14-B according to US 7,687,423 B2

In a 2 l plastic beaker 91 g fumed silica (CAB-O-SIL M5, Sigma-Aldrich) are provided. In a separate plastic beaker, 960 g of deionized water are weighed in, and 15.63 g of sodium hydroxide (NaOH, Sigma-Aldrich), 11.28 g of sodium aluminate (NaAlO$_2$, Sigma-Aldrich), and 12.65 g tetraethylammonium bromide (TEABr, Aldrich) are added und stirring and the mixture is further stirred until complete dissolution thereof is achieved. The solution is then added to the beaker containing the fumed silica under stirring for providing a viscous gel, which is further stirred for 2 h. The synthesis gel thus obtained (1.07 kg) displaying a molar composition of 0.2 $Na_2O$ : 0.051 $Al_2O_3$ : $SiO_2$ : 39.5 $H_2O$ : 0.045 TEABr is then distributed among several pressure tight vessels and then crystallized for 76 h at 150 °C under agitating at a stirring speed of 300 rpm. The resulting product is then filtered off as a solid, washed with deionized water, and dried, followed by a step of heating the solids under a nitrogen atmosphere with a heating rate of 2 °C per minute to 540 °C and calcining the material at said temperature for 2 h, after which calcination at that temperature is continued in air for an additional 5 h. The yield was 59.1 g.

According to the elemental analysis the resulting product had the following contents determined per 100 g substance of < 0.1 g carbon, 4.7 g aluminum, 2.8 g sodium and 38 g silicon.

The BET surface area was determined to be 416 $m^2$/g. The crystallinity of the product was measured to be 80%.

As taken from the X-ray diffraction pattern of the resulting product displayed in Figure 1, the zeolitic material obtained displays the MOR framework structure as the single crystalline phase. The average crystal size as calculated from calculated from the X-ray diffraction data was determined to be 47.5 nm, and the average crystal size along the 002 axis of the crystallites was determined to be 33 nm.

In a 2 liter stirring apparatus, 50 g of ammonium nitrate dissolved in 450 g of distilled water were placed as an aqueous solution (10 wt.-% $NH_4NO_3$), 50 g of the zeolitic material were added, and the resulting mixture was stirred for 2 h at 80°C. The zeolitic material was then filtered off and a new 10-wt.% aqueous solution containing 50 g of ammonium nitrate dissolved in 450 g of distilled water was then placed in the stirring apparatus to which the filtered off zeolitic material was added and the resulting mixture again stirred for 2h at 80°C. The zeolitic material was then filtered off and washed with distilled water until the wash water was free of nitrate. The washed material was then dried for 4 h at 120°C and subsequently calcined at 500°C in air for 5 h. The entire procedure was then repeated, affording 40.8 g of the H-form of the zeolitic material.

According to the elemental analysis, the resulting sample had the following contents determined per 100 g substance of 4.2 g aluminum, < 0.01 g sodium and 38 g silicon.

The BET surface area was determined to be 486 $m^2$/g. The crystallinity of the product was measured to be 71 %, and the average crystal size as calculated from calculated from the X-ray diffraction data was determined to be 47 nm, and the average crystal size along the 002 axis of the crystallites was determined to be 33 nm.

**Comparative Example 1:** Synthesis of H-Mordenite

In a 5 l plastic beaker 120 g fumed silica (CAB-O-SIL M5, Sigma-Aldrich) are suspended in 900 g deionized water. To this suspension a mixture of 52.04 g tetraethylammonium bromide (TEABr, Aldrich) in 161.7 g deionized water is added. The resulting mixture is agitated for 1 h at a stirring speed of 200 rpm. Then, a mixture of 36.5 g sodium hydroxide flakes (NaOH, Sigma-Aldrich) in 161.7 g deionized water is added. The resulting mixture is then agitated for 1.5 h at a stirring speed of 300 rpm. Subsequently, 188.6 g deionized water are added and then a mixture of 15.66 g sodium aluminate ($NaAlO_2$, Sigma-Aldrich) in 188.6 g deionized water. The resulting mixture is then agitated for 1 h at a stirring speed of 200 rpm. The pH value of the mixture was determined to be 12.5. A gel is formed which aged over night.

The synthetic gel displaying a molar composition of 0.28 $Na_2O$ : 0.048 $Al_2O_3$ : $SiO_2$ : 44.5 $H_2O$ : 0.13 TEABr is then crystallized in a pressure tight vessel for 84 h at 170 °C under agitating at a stirring speed of 250 rpm. Then, the resulting product is filtered off as a solid and washed with deionized water until the electrical conductance of the washing water reaches a value lower than 150 $\mu$S. The solids are then dried in air at 90 °C for 12 h. Subsequently, the solids are heated in air to 90 °C with a heating rate of 3.5 °C per minute and then left at said temperature for 2 h. Then the solids are heated to 120 °C with a heating rate of 1.5 °C per minute and then left at said temperature for 2 h. Then the solids are heated to 550 °C with a heating rate of 4.5 °C/min and left at said temperature for 12 h. The yield was 66 g.

According to the elemental analysis the resulting product had the following contents determined per 100 g substance of 0.1 g carbon, 5.0 g aluminum, 3.2 g sodium and 37 g silicon.

The BET surface area was determined to be 382 $m^2$/g. The crystallinity of the product was measured to be 86%.

As taken from the X-ray diffraction pattern of the resulting product, the zeolitic material obtained displays the MOR framework structure as the single crystalline phase, wherein the average crystal size along the 002 axis of the crystallites as calculated from the X-ray diffraction data was determined to be 58 nm.

In a 2 liter stirring apparatus, 50 g of ammonium nitrate dissolved in 450 g of distilled water were placed as an aqueous solution (10 wt.-% $NH_4NO_3$), 50 g of the zeolitic material were added, and the resulting mixture was stirred for 2 h at 80°C. The zeolitic material was then filtered off and a new 10-wt.% aqueous solution containing 50 g of ammonium nitrate dissolved in 450 g of distilled water was then placed in the stirring apparatus to which the filtered off zeolitic material was added and the resulting mixture again stirred for 2h at 80°C. The zeolitic material was then filtered off and washed with distilled water until the wash water was free of nitrate. The washed material was then dried for 5 h at 120°C and subsequently calcined at 500°C for 5 h with a heating rate of 2°C/min. The entire procedure was then repeated, affording 43.7 g of the H-form of the zeolitic material.

According to the elemental analysis, the resulting sample had the following contents determined per 100 g substance of <0.1 g carbon, 4.9 g aluminum, 0.06 g sodium and 38 g silicon.

The BET surface area was determined to be 432 $m^2/g$.

**Comparative Example 2:** Synthesis of H-Mordenite

In a 5 l plastic beaker 90 g fumed silica (CAB-O-SIL M5, Sigma-Aldrich) are suspended in 675 g deionized water. To this suspension a mixture of 39.03 g tetraethylammonium bromide (TEABr, Aldrich) in 121.3 g deionized water is added. The resulting mixture is agitated for 1 h at a stirring speed of 200 rpm. Then, a mixture of 27.88 g sodium hydroxide flakes (NaOH, Sigma-Aldrich) in 121.3 g deionized water is added. The resulting mixture is then agitated for 1.5 h at a stirring speed of 300 rpm. Subsequently, a mixture of 11.75 g sodium aluminate ($NaAlO_2$, Sigma-Aldrich) in 141.45 g deionized water are added and then 33.86 g of 1,6-hexanediol. The resulting mixture is then agitated for 1 h at a stirring speed of 200 rpm. Finally, 150 g of n-decane were added and the pH value of the mixture was determined to be 10.2. A gel is formed which aged over night.

The synthetic gel displaying a molar composition of 0.28 $Na_2O$ : 0.048 $Al_2O_3$ : $SiO_2$ : 39.2 $H_2O$ : 0.19 1,6-hexanediol : 0.7 n-decane : 0.124 TEABr is then crystallized in a pressure tight vessel for 120 h at 170 °C under agitating at a stirring speed of 250 rpm. Then, the resulting product is filtered off as a solid and washed with deionized water until the electrical conductance of the washing water reaches a value lower than 150 $\mu$S. The solids are then dried in air at 90 °C for 12 h. Subsequently, the solids are heated in air to 90 °C with a heating rate of 3.5 °C per minute and then left at said temperature for 2 h. Then the solids are heated to 120 °C with a heating rate of 1.5 °C per minute and then left at said temperature for 2 h. Then the solids are heated to 550 °C with a heating rate of 4.5 °C/min and left at said temperature for 12 h. The yield was 56 g.

According to the elemental analysis the resulting product had the following contents determined per 100 g substance of < 0.1 g carbon, 5.0 g aluminum, 3.3 g sodium and 37 g silicon.

The BET surface area was determined to be 398 $m^2/g$. The crystallinity of the product was measured to be 89%.

In a 2 liter stirring apparatus, 50 g of ammonium nitrate dissolved in 450 g of distilled water were placed as an aqueous solution (10 wt.-% $NH_4NO_3$), 50 g of the zeolitic material were added, and the resulting mixture was stirred for 2 h at 80°C. The zeolitic material was then filtered off and washed with distilled water until the wash water was free of nitrate. A new 10-wt.% aqueous solution containing 50 g of ammonium nitrate dissolved in 450 g of distilled water was then placed in the stirring apparatus to which the washed zeolitic material was added and the resulting mixture again stirred for 2h at 80°C. The zeolitic material was then filtered off and washed anew with distilled water until the wash water was free of nitrate. The washed material was then dried for 5 h at 120°C and subsequently calcined at 500°C for 5 h with a heating rate of 2°C/min. The entire procedure was then repeated, affording 45.7 g of the H-form of the zeolitic material.

According to the elemental analysis, the resulting sample had the following contents determined per 100 g substance of <0.1 g carbon, 5.3 g aluminum, < 0.01 g sodium and 39 g silicon.

The BET surface area was determined to be 440 $m^2/g$.

As taken from the X-ray diffraction pattern of the resulting product, the zeolitic material obtained displays the MOR framework structure as the single crystalline phase, wherein the average crystal size along the 002 axis of the crystallites as calculated from the X-ray diffraction data was determined to be 60 nm.

**Comparative Example 3:** Synthesis of H-Mordenite from commercial Na-MOR

In a 2 liter stirring apparatus, 200 g of ammonium chloride dissolved in 800 ml of distilled water were placed as an aqueous solution (20 wt.-% $NH_4Cl$), 100 g of Na-Mordenite (FM-8, Zeochem) were added, and the resulting mixture was stirred for 2 h at 100°C. The zeolitic material was then filtered off and washed with distilled water until the wash water was free of chloride. The washed material was then dried for 12 h at 120°C and subsequently calcined at 500°C for 5 h with a heating rate of 2°C/min. The procedure afforded 97.8 g of the H-form of the commercial zeolitic material.

According to the elemental analysis, the resulting sample had the following contents determined per 100 g substance of < 0.1 g carbon, 2.8 g aluminum, < 0.01 g sodium and 38 g silicon.

As calculated from the X-ray diffraction data of the commercial sample, the average crystal size along the 002 axis of the crystallites was determined to be 77 nm.

**Comparative Example 4:** Commercial Mordenite in the H-form

A commercial sample of H-Mordenite (TZM-1013, Tricat) was directly employed as Comparative Example 4.

According to the elemental analysis, the sample had the following contents determined per 100 g substance of < 0.1 g carbon, 5.4 g aluminum, 0.03 g sodium and 36 g silicon.

As calculated from the X-ray diffraction data of the commercial sample, the average crystal size was determined to be 71 nm, and the average crystal size along the 002 axis of the crystallites was determined to be 99 nm.

**Comparative Example 5:** Commercial Mordenite in the H-form

A further commercial sample of H-Mordenite (MOR-1501, Novel) was directly employed as Comparative Example 5.

According to the elemental analysis, the sample had the following contents determined per 100 g substance of 5.1 g aluminum and 40 g silicon.

As calculated from the X-ray diffraction data of the commercial sample, the average crystal size was determined to be 91.5 nm, and the average crystal size along the 002 axis of the crystallites was determined to be 83 nm.

**Example 4:** Catalyst Testing

Into a carrier gas stream consisting of nitrogen and specific amounts of methane (as internal standard), hydrogen, ammonia, and monoethanolamine (MEOA) are evaporated at a temperature according to their partial pressures. Ammonia is evaporated in a first evaporator whereas MEOA is evaporated in a second evaporator downstream. Afterwards the resultant gas vapor stream is heated to 200°C.

The zeolitic materials to be tested were respectively admixed with 3 wt.-% graphite and homogenized by shaking and mixing, if necessary using a mortar and pestle. The homogenized mixture is then pelletized using a 13 mm diameter pelletizing tool set applying 10-40 kN of force depending on the zeolite in order to obtain stable pellets and thus a stable target fraction, wherein the pellets obtained are 2-3 mm in height and have a diameter of 13mm. The pellets thus obtained were then precrushed with mortar and pestle and sieved through a 1000 $\mu$m analytical sieve. Crushing and sieving was repeated for obtaining the desired target fraction having a particle diameter in the range of from 315-500 $\mu$m using suitable analytical sieves and a pestle, and wherein the fines (<315 $\mu$m) were removed by sieving on a sieving tool (e.g. Retsch AS 200) or by sieving manually.

This gas vapor stream is fed to a reactor filled with 1 cm$^3$ of catalyst particles that are of the size in the range of 315 - 500 $\mu$m. The catalyst bed has a diameter of 4 mm and a length of 80 mm. Due to the low diameter of the catalyst bed it is isothermal. Before the catalyst bed the gas vapor stream is heated to the reaction temperature by passing it through an inert bed. Both the catalyst bed and the inert bed are heated externally to the reaction temperature. Downstream to the catalyst bed the product stream is diluted and cooled to 250°C. Further downstream its composition is measured by an online-GC.

Results were calculated by referencing the ratio of educt to internal standard (IS) to the same ratio as obtained by analyzing the gas vapor stream from a by-pass tubing. Thus undetected products (high-boilers, coke) are taken into account as well. The following formulas give the detailed procedure:

$$\text{Conversion:} \quad X(\text{educt})= 1-c(\text{educt})/c(\text{IS})/(c(\text{educt\_by-pass})/c(\text{IS-by-pass}))$$

$$\text{Yields:} \quad Y(\text{product})= c(\text{product})/c(\text{IS})/(c(\text{educt\_by-pass})/c(\text{IS-by-pass}))$$

$$\text{Selectivities:} \quad S(\text{product})=Y(\text{product})/X(\text{educt})$$

For the standard experiment the following testing conditions were chosen: gas hourly space velocity (GHSV) of 5000 h$^{-1}$ with MEOA-concentration of 1 Vol-%. Apart from the main educt MEOA the gas stream consisted of 40 vol.-% ammonia, 20 vol.-% hydrogen and 1 vol.-% methane as internal standard with nitrogen as balance. The catalysts were heated in nitrogen to the reaction temperature of 300°C and then the gas feed was switched to testing conditions. The results obtained from catalytic testing performed on Examples 1-3 and Comparative Examples 1-5 are displayed in Table 1 below, wherein the yield of ethylene diamine and the conversion rate of MEOA are respectively shown in %, as well as the amounts of diethylenetriamine (DETA), aminoethylethanolamine (AEEA), piperazine (PIP), 1-(2-aminoethyl)piperazine (AE-PIP), and 1,4-diazabicyclo[2.2.2]octane (DABCO) generated in the reaction in %. As regards the results obtained for Examples 1-3, values are indicated as obtained from 2 different runs, respectively.

Table 1: Results from catalytic testing of Examples 1-3 and Comparative Examples 1-5.

| Example | average 002 crystal plane dimension [nm] | EDA Yield [%] | DETA Yield [%] | AEEA Yield [%] | PIP Yield [%] | AE-PIP Yield [%] | DABCO Yield [%] | MEOA conversion [%] |
|---|---|---|---|---|---|---|---|---|
| Ex. 3 | 33 | 31.9 | 0.4 | 1.6 | 2.2 | 3.2 | 1.8 | 53.0 |
| Ex. 1 | 46 | 32.1 | 0.5 | 2.1 | 1.1 | 2.1 | 1.2 | 48.3 |
| Ex. 2 | 46 | 35.2 | 0.7 | 1.8 | 1.7 | 2.8 | 1.5 | 52.5 |
| Comp. Ex. 1 | 58 | 20.8 | <0.1 | 2.4 | 0.7 | 1.4 | 0.8 | 34.3 |
| Comp. Ex. 2 | 60 | 4.0 | 1.1 | 1.2 | 5.3 | 7.2 | 3.7 | 52.6 |
| Comp. Ex. 5 | 83 | 28.4 | 0.7 | 2.5 | 1.3 | 2.7 | 1.2 | 47.4 |
| Comp. Ex. 3 | 77 | 6.9 | <0.1 | 1.8 | 0.6 | 1.3 | 0.6 | 14.6 |
| Comp. Ex. 4 | 99 | 9.6 | <0.1 | 1.4 | 0.6 | 1.4 | 0.5 | 15.5 |

Thus, as may be taken from the results displayed in Table 1, all of the inventive samples having an average 002 crystal plane dimension of less than 55 nm display a clearly superior performance in the catalytic amination of MEOA to EDA, both in view of MEOA conversion, as well as with respect to the yield in EDA which may be realized. As may be taken from the results obtained for Example 2 compared to those obtained for Example 1, the superior performance may be further increased by ion exchange of the H-form with copper.

Therefore, as demonstrated in the foregoing, it has surprisingly been found that a zeolitic material having the MOR framework structure and which displays an average 002 crystal plane dimension of less than 55 nm not only displays a considerably improved catalytic activity in the amination of MEOA, but furthermore displays a highly improved selectivity as may be observed from the results for the yield in EDA achieved by the inventive samples. Consequently, it has quite unexpectedly been found that a highly improved process for the amination of MEOA to EDA may be obtained by using a zeolitic material having the MOR framework structure displaying an average 002 crystal plane dimension of less than 55 nm, wherein said effect may be further increased by ion exchange of the H-form with copper.

List of the cited prior art references:

- WO 2014/135662 A

- US 7,605,295

- US 7,687,423 B2

- Grundner, Sebastian et al. in Nat. Commun. 2015, Vol. 6, , article number 7546

- US 4,918,233

- CN 1962058 A

- JP H0687797 A

- JP H07247245 A

- CN 101215239 B

- CN 101406845 A

- CN 102974393 A

- CN 103007984 A

- CN102233272A

- CN102190588A

- inaugural thesis "Heterogeneous Transition Metal Catalyzed Amination of Aliphatic Diols" from Achim Fischer, Diss. ETH No 12978, 1998

- WO 2009/083580 A1

- US 4918233

- CN 101215239 A

**Claims**

1. A process for the conversion of 2-aminoethanol to ethane-1,2-diamine and/or linear polyethylenimines of the formula $H_2N\text{-}[CH_2CH_2NH]_n\text{-}CH_2CH_2NH_2$ wherein $n \geq 1$ comprising

   (i) providing a catalyst comprising a zeolitic material having the MOR framework structure comprising $YO_2$ and $X_2O_3$, wherein Y is a tetravalent element and X is a trivalent element;
   (ii) providing a gas stream comprising 2-aminoethanol and ammonia;
   (iii) contacting the catalyst provided in (i) with the gas stream provided in (ii) for converting 2-aminoethanol to ethane-1,2-diamine and/or linear polyethylenimines,

   wherein the average particle size of the zeolitic material along the 002 axis of the crystallites is in the range of from $5 \pm 1$ nm to $55 \pm 8$ nm as determined by powder X-ray diffraction.

2. The process of claim 1, wherein the average particle size of the primary crystallites of the zeolitic material as determined by powder X-ray diffraction is in the range of from $5 \pm 1$ nm to $100 \pm 15$ nm.

3. The process of claim 1 or 2, wherein the gas stream provided in (ii) and contacted with the catalyst in (iii) contains 2-aminoethanol in an amount in the range of from 0.1 to 10 vol.-%.

4. The process of any of claims 1 to 3, wherein the gas stream provided in (ii) and contacted with the catalyst in (iii) contains ammonia in an amount in the range of from 5 to 90 vol.-%.

5. The process of any of claims 1 to 4, wherein the gas stream provided in (ii) and contacted with the catalyst in (iii) further contains hydrogen in an amount in the range of from 0.1 to 70 vol.-%.

6. The process of any of claims 1 to 5, wherein the gas stream provided in (ii) and contacted with the catalyst in (iii) contains 1 vol.-% or less of hydrogen.

7. The process of any of claims 1 to 6, wherein the gas stream provided in (ii) and contacted with the catalyst in (iii) contains $H_2O$ in an amount of 5 vol.-% or less.

8. The process of any of claims 1 to 7, wherein the gas stream provided in (ii) is heated to a temperature in the range of from 120 to 600°C, prior to contacting with the catalyst in (iii) at that temperature.

9. The process of claim 1 to 8, wherein Y is selected from the group consisting of Si, Sn, Ti, Zr, Ge, and mixtures of two or more thereof.

10. The process of any of claims 1 to 9, wherein X is selected from the group consisting of Al, B, In, Ga, and mixtures of two or more thereof.

11. The process of any of claims 1 to 10, wherein the zeolitic material having the MOR framework structure is in the H-form and contains protons as extra-framework ions, wherein 0.1 wt.-% or less of the extra-framework ions are metal cations, calculated as the element and based on 100 wt.-% of $YO_2$ contained in the zeolitic material.

12. The process of any of claims 1 to 11, wherein the zeolitic material contains substantially no Na.

13. The process of any of claims 1 to 12, wherein the zeolitic material having the MOR framework structure is prepared by a process comprising

(1) preparing a mixture comprising at least one source of $YO_2$, at least one source of $X_2O_3$, and comprising one or more organotemplates as structure directing agent and/or comprising seed crystals;
(2) crystallizing the mixture prepared in (i) for obtaining a zeolitic material having the MOR framework structure;
(3) optionally isolating the zeolitic material obtained in (2);
(4) optionally washing the zeolitic material obtained in (2) or (3);
(5) optionally drying and/or calcining the zeolitic material obtained in (2), (3), or (4);
(6) optionally subjecting the zeolitic material obtained in (2), (3), (4), or (5) to an ion-exchange procedure, wherein extra-framework ions contained in the zeolitic material are ion-exchanged against $H^+$;
(7) optionally subjecting the zeolitic material obtained in (2), (3), (4), (5), or (6) to an ion-exchange procedure, wherein extra-framework ions contained in the zeolitic material are ion-exchanged against one or more metal ions M selected from the group consisting of alkaline earth metals and/or transition metals;
(8) optionally drying and/or calcining the zeolitic material obtained in (7).

14. The process of claim 13, wherein in (6) the step of subjecting the zeolitic material to an ion-exchange procedure includes the steps of

(6.a) subjecting the zeolitic material obtained in (2), (3), (4), or (5) to an ion-exchange procedure, wherein extra-framework ions contained in the zeolitic material are ion-exchanged against $NH_4^+$;
(6.b) calcining the ion-exchanged zeolitic material obtained in (6.a) for obtaining the H-form of the zeolitic material.

15. The process of any of claims 1 to 14, wherein 2-aminoethanol comprised in the gas stream obtained in (iii) is separated from said gas stream and recycled to (ii).

**Patentansprüche**

1. Verfahren zur Umwandlung von 2-Aminoethanol in Ethan-1,2-diamin und/oder lineare Polyethylenimine der Formel $H_2N-[CH_2CH_2NH]_n-CH_2CH_2-NH_2$, wobei $n \geq 1$, das Folgendes umfasst:

(i) Bereitstellen eines Katalysators, der ein zeolithisches Material mit der MOR-Gerüststruktur, die $YO_2$ und $X_2O_3$ umfasst, aufweist, wobei Y für ein vierwertiges Element steht und X für ein dreiwertiges Element steht;
(ii) Bereitstellen eines Gasstroms, der 2-Aminoethanol und Ammoniak umfasst;
(iii) Inkontaktbringen des in (i) bereitgestellten Katalysators mit dem in (ii) bereitgestellten Gasstrom zur Umwandlung von 2-Aminoethanol in Ethan-1,2-diamin und/oder lineare Polyethylenimine,

wobei die durchschnittliche Teilchengröße des zeolithischen Materials entlang der 002-Achse der Kristallite gemäß Bestimmung durch Pulverröntgenbeugung im Bereich von 5 ± 1 nm bis 55 ± 8 nm liegt.

2. Verfahren nach Anspruch 1, bei dem die durchschnittliche Teilchengröße der Primärkristallite des zeolithischen Materials gemäß Bestimmung durch Pulverröntgenbeugung im Bereich von 5 ± 1 nm bis 100 ± 15 nm liegt.

3. Verfahren nach Anspruch 1 oder 2, bei dem der in (ii) bereitgestellte und in (iii) mit dem Katalysator in Kontakt gebrachte Gasstrom 2-Aminoethanol in einer Menge im Bereich von 0,1 bis 10 Vol.-% enthält.

4. Verfahren nach einem der Ansprüche 1 bis 3, bei dem der in (ii) bereitgestellte und in (iii) mit dem Katalysator in Kontakt gebrachte Gasstrom Ammoniak in einer Menge im Bereich von 5 bis 90 Vol.-% enthält.

5. Verfahren nach einem der Ansprüche 1 bis 4, bei dem der in (ii) bereitgestellte und in (iii) mit dem Katalysator in Kontakt gebrachte Gasstrom ferner Wasserstoff in einer Menge im Bereich von 0,1 bis 70 Vol.-% enthält.

6. Verfahren nach einem der Ansprüche 1 bis 5, bei dem der in (ii) bereitgestellte und in (iii) mit dem Katalysator in Kontakt gebrachte Gasstrom 1 Vol.-% oder weniger Wasserstoff enthält.

7. Verfahren nach einem der Ansprüche 1 bis 6, bei dem der in (ii) bereitgestellte und in (iii) mit dem Katalysator in Kontakt gebrachte Gasstrom $H_2O$ in einer Menge von 5 Vol.-% oder weniger enthält.

8. Verfahren nach einem der Ansprüche 1 bis 7, bei dem der in (ii) bereitgestellte Gasstrom auf eine Temperatur im Bereich von 120 bis 600 °C erhitzt wird, bevor er in (iii) bei dieser Temperatur mit dem Katalysator in Kontakt gebracht wird.

9. Verfahren nach Anspruch 1 bis 8, bei dem Y aus der Gruppe bestehend aus Si, Sn, Ti, Zr, Ge und Mischungen von zwei oder mehr davon ausgewählt ist.

10. Verfahren nach einem der Ansprüche 1 bis 9, bei den X aus der Gruppe bestehend aus Al, B, In, Ga und Mischungen von zwei oder mehr davon ausgewählt ist.

11. Verfahren nach einem der Ansprüche 1 bis 10, bei dem das zeolithische Material mit der MOR-Gerüststruktur in der H-Form vorliegt und Protonen als Extra-Gerüst-Ionen enthält, wobei 0,1 Gew.-% oder weniger der Extra-Gerüst-Ionen Metallkationen sind, berechnet als Element und bezogen auf 100 Gew.-% des in dem zeolithischen Material enthaltenen $YO_2$.

12. Verfahren nach einem der Ansprüche 1 bis 11, bei dem das zeolithische Material weitgehend kein Na enthält.

13. Verfahren nach einem der Ansprüche 1 bis 12, bei dem das zeolithische Material mit der MOR-Gerüststruktur durch ein Verfahren hergestellt wird, das Folgendes umfasst:

(1) Herstellen einer Mischung, die mindestens eine Quelle von $YO_2$, mindestens eine Quelle von $X_2O_3$ und ein oder mehrere Organotemplate als Strukturbildner und/oder Impfkristalle umfasst;
(2) Kristallisieren der in (i) hergestellten Mischung zum Erhalt eines zeolithischen Materials mit der MOR-Gerüststruktur;
(3) gegebenenfalls Isolieren des in (2) erhaltenen zeolithischen Materials;
(4) gegebenenfalls Waschen des in (2) bzw. (3) erhaltenen zeolithischen Materials;
(5) gegebenenfalls Trocknen und/oder Calcinieren des in (2), (3) bzw. (4) erhaltenen zeolithischen Materials;
(6) gegebenenfalls Durchführen eines Ionenaustauschprozesses an dem in (2), (3), (4) bzw. (5) erhaltenen zeolithischen Material, wobei in dem zeolithischen Material enthaltene Extra-Gerüst-Ionen gegen $H^+$ ausgetauscht werden;
(7) gegebenenfalls Durchführen eines Ionenaustauschprozesses an dem in (2), (3), (4), (5) bzw. (6) erhaltenen zeolithischen Material, wobei in dem zeolithischen Material enthaltene Extra-Gerüst-Ionen gegen ein oder mehrere Metallionen M aus der Gruppe bestehend aus Erdalkalimetallen und/oder Übergangsmetallen ausgetauscht werden;
(8) gegebenenfalls Trocknen und/oder Calcinieren des in (7) erhaltenen zeolithischen Materials.

14. Verfahren nach Anspruch 13, bei dem in (6) der Schritt des Durchführens eines Ionenaustauschprozesses an dem zeolithischen Material die folgenden Schritte umfasst:

(6.a) Durchführen eines Ionenaustauschprozesses an dem in (2), (3), (4) bzw. (5) erhaltenen zeolithischen Material, wobei in dem zeolithischen Material enthaltene Extra-Gerüst-Ionen gegen $NH_4^+$-Ionen ausgetauscht werden;
(6.b) Calcinieren des in (6.a) erhaltenen ionenausgetauschtem zeolithischen Materials zum Erhalt der H-Form des zeolithischen Materials.

15. Verfahren nach einem der Ansprüche 1 bis 14, bei dem man in dem in (iii) erhaltenen Gasstrom enthaltenes 2-Aminoethanol aus dem Gasstrom abtrennt und zu (ii) zurückführt.

**Revendications**

1. Procédé pour la conversion de 2-aminoéthanol en éthane-1,2-diamine et/ou en polyéthylèneimines linéaires de la formule $H_2N\text{-}[CH_2CH_2NH]_n\text{-}CH_2CH_2\text{-}NH_2$, $n \geq 1$ comprenant

   (i) la mise à disposition d'un catalyseur comprenant un matériau zéolithique possédant la structure de cadre MOR comprenant $YO_2$ et $X_2O_3$, Y étant un élément tétravalent et X étant un élément trivalent ;
   (ii) la mise à disposition d'un flux de gaz comprenant du 2-aminoéthanol et de l'ammoniac ;
   (iii) la mise en contact du catalyseur mis à disposition en (i) avec le flux de gaz mis à disposition en (ii) pour convertir le 2-aminoéthanol en éthane-1,2-diamine et/ou en polyéthylèneimines linéaires,

   la taille moyenne de particule du matériau zéolithique le long de l'axe 002 des cristallites étant dans la plage allant de $5 \pm 1$ nm à $55 \pm 8$ nm telle que déterminée par diffraction des rayons X sur poudre.

2. Procédé selon la revendication 1, la taille moyenne de particule des cristallites primaires du matériau zéolithique telle que déterminée par diffraction des rayons X sur poudre étant dans la plage allant de $5 \pm 1$ nm à $100 \pm 15$ nm.

3. Procédé selon la revendication 1 ou 2, le flux de gaz mis à disposition en (ii) et mis en contact avec le catalyseur en (iii) contenant du 2-aminoéthanol en une quantité dans la plage allant de 0,1 à 10 % en volume.

4. Procédé selon l'une quelconque des revendications 1 à 3, le flux de gaz mis à disposition en (ii) et mis en contact avec le catalyseur en (iii) contenant de l'ammoniac en une quantité dans la plage allant de 5 à 90 % en volume.

5. Procédé selon l'une quelconque des revendications 1 à 4, le flux de gaz mis à disposition en (ii) et mis en contact avec le catalyseur en (iii) contenant en outre de l'hydrogène en une quantité dans la plage allant de 0,1 à 70 % en volume.

6. Procédé selon l'une quelconque des revendications 1 à 5, le flux de gaz mis à disposition en (ii) et mis en contact avec le catalyseur en (iii) contenant 1 % en volume ou moins d'hydrogène.

7. Procédé selon l'une quelconque des revendications 1 à 6, le flux de gaz mis à disposition en (ii) et mis en contact avec le catalyseur en (iii) contenant $H_2O$ en une quantité de 5 % en volume ou moins.

8. Procédé selon l'une quelconque des revendications 1 à 7, le flux de gaz mis à disposition en (ii) étant chauffé à une température dans la plage allant de 120 à 600 °C, avant la mise en contact avec le catalyseur en (iii) à cette température.

9. Procédé selon les revendications 1 à 8, Y étant choisi dans le groupe constitué par Si, Sn, Ti, Zr, Ge, et des mélanges de deux ou plus de ceux-ci.

10. Procédé selon l'une quelconque des revendications 1 à 9, X étant choisi dans le groupe constitué par Al, B, In, Ga, et des mélanges de deux ou plus de ceux-ci.

11. Procédé selon l'une quelconque des revendications 1 à 10, le matériau zéolithique possédant la structure de cadre MOR étant sous la forme H et contenant des protons en tant qu'ions externes au cadre, 0,1 % en poids ou moins des ions externes au cadre étant des cations métalliques, calculé comme l'élément et basé sur 100 % en poids de $YO_2$ contenu dans le matériau zéolithique.

12. Procédé selon l'une quelconque des revendications 1 à 11, le matériau zéolithique ne contenant sensiblement pas de Na.

13. Procédé selon l'une quelconque des revendications 1 à 12, le matériau zéolithique possédant la structure de cadre MOR étant préparé par un procédé comprenant

    (1) la préparation d'un mélange comprenant au moins une source de $YO_2$, au moins une source de $X_2O_3$, et comprenant une ou plusieurs matrices organiques en tant qu'agent de direction de structure et/ou comprenant des germes cristallins ;
    (2) la cristallisation du mélange préparé en (1) pour l'obtention d'un matériau zéolithique possédant la structure de cadre MOR ;

(3) éventuellement l'isolement du matériau zéolithique obtenu en (2) ;

(4) éventuellement le lavage du matériau zéolithique obtenu en (2) ou (3) ;

(5) éventuellement le séchage et/ou la calcination du matériau zéolithique obtenu en (2), (3), ou (4) ;

(6) éventuellement la soumission du matériau zéolithique obtenu en (2), (3), (4), ou (5) à une procédure d'échange d'ions, des ions externes au cadre contenus dans le matériau zéolithique étant soumis à un échange d'ions contre $H^+$ ;

(7) éventuellement la soumission du matériau zéolithique obtenu en (2), (3), (4), (5), ou (6) à une procédure d'échange d'ions, des ions externes au cadre contenus dans le matériau zéolithique étant soumis à un échange d'ions contre un ou plusieurs ions métalliques M choisis dans le groupe constitué par des métaux alcalino-terreux et/ou des métaux de transition ;

(8) éventuellement le séchage et/ou la calcination du matériau zéolithique obtenu en (7) .

14. Procédé selon la revendication 13, dans lequel en (6) l'étape de soumission du matériau zéolithique à une procédure d'échange d'ions comporte l'étape de

(6.a) soumission du matériau zéolithique obtenu en (2), (3), (4), ou (5) à une procédure d'échange d'ions, des ions externes au cadre contenus dans le matériau zéolithique étant soumis à un échange d'ions contre $NH_4^+$ ;

(6.b) calcination du matériau zéolithique soumis à un échange d'ions obtenu en (6.a) pour l'obtention de la forme H du matériau zéolithique.

15. Procédé selon l'une quelconque des revendications 1 à 14, le 2-aminoéthanol compris dans le flux de gaz obtenu en (iii) étant séparé dudit flux de gaz et recyclé vers (ii).

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014135662 A
- US 7605295 B
- US 7687423 B2
- US 4918233 A
- CN 1962058 A
- JP H0687797 A
- JP H07247245 A
- CN 101215239 B
- CN 101406845 A
- CN 102974393 A
- CN 103007984 A
- CN 102233272 A
- CN 102190588 A
- WO 2009083580 A1
- CN 101215239 A
- US 7605295 B1

### Non-patent literature cited in the description

- **GRUNDNER ; SEBASTIAN et al.** *Nat. Commun,* 2015, vol. 6 (7546
- **ACHIM FISCHER.** *Heterogeneous Transition Metal Catalyzed Amination of Aliphatic Diols,* 1998
- **ACHIM FISCHER.** Heterogeneous Transition Metal Catalyzed Amination of Aliphatic Diols. *inaugural thesis,* 1998